# EUROPEAN PATENT APPLICATION

(11) **EP 4 825 393 A1**
(43) Date of publication of application: **21.10.2026**
(21) Application number: 25171431.7
(22) Date of filing: 17.04.2025
(51) Int. Cl.: A61K 38/48, A61K 31/352, A61K 31/7048, A61K 36/88, A61P 9/12, A61P 9/14, A23L 33/105

(54) **COMPOSITION AND USES THEREOF**

(71) Applicant: Alfasigma S.p.A., 40133 Bologna (IT)
(72) Inventor: FIORE, Walter, 24126 Bergamo (BG) (IT)
(74) Representative: Trillat, Anne-Cecile

(57) **Abstract**

The invention refers to a composition comprising diosmin, hesperidin, *Ruscus aculeatus* extract, *Ananas comosus* extract, and bromelain. Preferably said composition is used in the treatment and/or prevention of a venous disease.

## Description

### Technical Field of the invention

The present invention relates to a composition comprising diosmin, hesperidin, *Ruscus* extract, *Ananas* extract and bromelain. The composition is used in the treatment and/or prevention of a venous disease, in particular a chronic venous disease.

### Background of the invention

Endothelial cells (EC) line blood vessels are crucial for maintaining health and preventing cardiovascular, metabolic, and pulmonary diseases. They regulate blood vessel functions, inflammation, oxidation, and clotting by sensing blood flow through specific receptors on their surfaces. Alterations in these receptors can disrupt EC function, leading to inflammation, increased cell adhesion, and various complications. When ECs do not function properly, they can result in inflammation, abnormal cell growth, and blood clotting [1]. The endothelium is involved in many homeostatic functions, playing a vital role in influencing the tone of blood vessels through the generation and secretion of vasodilatory substances, which include prostaglandins, nitric oxide (NO), and hyperpolarisation factors. These processes are essential for maintaining optimal vascular tone, particularly in pathological challenges [3]. Indeed, the dysregulation of vascular tone attributed to endothelial cells is characterised by reduced bioavailability of NO, which positively influences the endothelial expression of adhesion molecules, consequently facilitating the recruitment of immune cells to the vascular wall and stimulating the activation of smooth muscle cells, thus promoting their proliferation and migration [4]. Additionally, dysfunctional endothelium plays a significant role in perpetuating the inflammatory cascade, leading to pathological changes in the veins and exacerbation of venous disease [5], in particular chronic venous disease.

Venous diseases or venous insufficiency, in particular chronic venous disease (CVD) of the lower extremities, constitute a significant global health concern, impacting approximately 5 to 33 percent of the adult population. Chronic venous disease (CVD) is defined as "(any) morphological and functional abnormalities of the venous system of long duration manifested either by symptoms and/or signs indicating the need for investigation and/or care". Most common CVD symptoms include pain, heaviness, feeling of swelling, itching of the skin, cramps and fatigue which are exacerbated by prolonged standing or sitting.

The clinical presentations of CVD may range from relatively benign cosmetic issues to more severe complications, including venous ulcers, thrombus formation, painful oedema, swelling, and distal venous hypertension. Traditional therapeutic approaches to address CVD predominantly encompass anticoagulants and compression devices; however, these interventions focus on alleviating symptoms rather than guaranteeing optimal valve functionality [6]. It is estimated that 17% of males and 40% of females may encounter venous insufficiency at some point in their lives, attributable to augmented vascular pressure in the lower extremities, which results in heightened capillary permeability and subsequent fluid loss within the tissues [7]. Individuals exhibiting venous hypertension, which may arise due to various predisposing factors, including advanced age, obesity, prior pregnancies, familial predisposition, and/or environmental or occupational influences, face an elevated risk of developing cardiovascular pathologies. In most instances, this condition is correlated with dysfunctional venous valves and venous reflux; however, it may also stem from antecedent venous obstruction. This pathological condition may be due to phenomena attributable to venous hypertension and venous stasis, endothelial dysfunction and the expression of inflammatory mediators, especially leukocyte adhesion, leading to an imbalance in the microcirculation and a reduction in tissue oxygenation [10].

The normal venous system depends on the integrity of valves, vein walls and hemodynamics of venous blood flow. These components are interdependent and the disruption of one affects the integrity of the others. The secondary alterations further affect the initial event, triggering a vicious circle. Valve and vein wall alterations are the hypothetical primary cause of CVD. Key roles are played by venous hypertension, stasis, altered shear stress, and inflammation and endothelial dysfunction. Shear stress can promote the release of factors that reduce inflammation and the formation of reactive free radicals. By contrast, low or zero shear stress, disturbed or even turbulent flow, and especially reversal of the direction of flow, all promote an inflammatory and thrombotic phenotype.

The Clinical Etiology Anatomy Pathophysiology (CEAP) classification is an internationally accepted standard for describing patients with chronic venous disorders [61]. The C designation include the following: C0 - No visible or palpable signs of venous disease; C1 - Telangiectasias or reticular veins; C2 - Varicose veins; C2r - Recurrent varicose veins; C3 - Edema; C4 - Changes in skin and subcutaneous tissue secondary to CVD; C4a - Pigmentation or eczema; C4b - Lipodermatosclerosis or atrophie blanche; C4c - Corona phlebectatica; C5 - Healed ulcer; C6 - Active venous ulceration; C6r - Recurrent venous ulceration.

Each clinical class sub-characterized by a subscript indicating the presence (symptomatic, s) or absence (asymptomatic, a) of symptoms attributable to venous disease.

Clinical symptoms and signs indicate inflammation due to this condition, which activates inflammatory pathways. The endothelium and glycocalyx help to detect changes in stress and activate leukocytes, causing vein damage and inflammation in the tissue, where unbalanced cytokine and protein production leads to varicose veins and ulcers [50]. In addition, the endothelial layer is responsible for maintaining vascular tone and the permeability of the vessel wall, so its impairment could promote adverse events leading to increased vascular tone [14,51]. Recently, it has been hypothesized that a potential molecular aetiology of this condition may be attributed to the elevated expression of matrix metalloproteinases (MMPs), which can facilitate venous wall dilation. Various factors, including venous hydrostatic pressure, hypoxia, tissue metabolites, and inflammatory processes, modulate the expression and activity of MMPs. In turn, MMPs enhance the proteolytic degradation of multiple protein substrates within the extracellular matrix, with a particular emphasis on collagen and elastin, compromising the structural integrity of the venous wall. [12]. The histopathological examination of varicose veins (VV) tissue reveals an absence of clearly defined vascular layers, and no discernible demarcations between the tunica intima, tunica media, and adventitia. [13]. VV tissue sections may also show focal thickening of the tunica intima and increased thickening of the tunica media with fragmented elastin fibres [14]. In this context, vascular endothelial growth factor (VEGF) exhibits a significant correlation with the production of matrix metalloproteinase-9 (MMP-9) and the recruitment of leukocytes to the locus of endothelial injury. Moreover, VEGF is pivotal in preserving the vascular endothelium's structural integrity during angiogenesis while simultaneously disrupting vascular wall stability and cellular homeostasis by enhancing endothelial permeability. Additionally, it facilitates the activation of endothelial nitric oxide synthase (eNOS), which induces the dilation of venous vessels. Dysregulated VEGF production may be a prognostic biomarker for vascular pathologies [15]. VEGF is known to stimulate the expression of eNOS in endothelial cells and thus causes a steady increase in NO [16]. In addition, the impairment of endothelium-derived NO synthesis associated with endothelial dysfunction causes or increases several adverse effects, including vasoconstriction and thrombosis, as well as the expression of pro-inflammatory cytokines and chemokines [51]. For this, some naturally derived substances, such as flavonoids, have been proposed to stimulate endothelial cells to release NO by mediating the increase in intracellular calcium levels and causing immediate vasodilation [10]. For instance, diosmin, frequently utilised with hesperidin (another flavonoid), is extensively employed in managing CVD. Empirical investigations have demonstrated that administering micronised flavonoids, such as micronised purified flavonoid fraction (MPFF), markedly alleviates symptoms associated with CVD, including pain, oedema, and a sensation of heaviness in the lower extremities. The micronisation process enhances bioavailability, thereby rendering therapeutic interventions more efficacious even at reduced dosages [17-19].

Nutritional or standard pharmaceutical (PS) supplements are available to support the management of CVD as it evolves. They can alleviate the signs and symptoms of CVD as an adjunctive treatment. Furthermore, patients using adjunctive medical management for CVD benefit from several products [46].

IT RM20120591 discloses a composition comprising diosmin, hesperidin, Rutin and Luteolin for use in increasing wellness of subjects affected by venous insufficiency and flebopathologies.

EP 2022345 discloses a food composition comprising hesperidin, isonarigin, eriocitrin and diosmin. The composition is useful for preventing, reducing or treating cardiovascular diseases and varicose veins, heavy legs or haemorrhoids.

IT BA20080014 discloses a topical composition comprising bromelain. Bromelain is used to reduce phlogosis, oedema and thrombus. In addition to bromelain, the composition may also comprise quercitin, hesperidin, *Vitis vinifera,* aescin, folic acid and/or menthol.

AT 501221 discloses a composition comprising at least one of aescin, rutoside, troxerutin, diosmin, hesperidin, gingko, *Vitis vinifera,* and/or *Ruscus aculeatus* to enhance blood circulation, in particular in the legs.

EP2353596 discloses a combination composition comprising L-carnitine or a derivative or a salt thereof, troxerutin, diosmin, hesperidin and its use for the prevention or treatment of diseases of the vein.

There is still the need to optimize suitable compositions to improve endothelium tone, integrity and response.

The present invention relates to a composition comprising diosmin, hesperidin, *Ruscus* extract, *Ananas* extract, and bromelain, in particular for use the treatment and/or prevention of vascular diseases.

### Summary of the invention

As outlined above, it has previously been found that the combination of diosmin and hesperidin is used in managing CVD. The present invention is based on a synergistic effect of the combination of diosmin, hesperidin, *Ruscus* extract, *Ananas* extract, and bromelain to enhance cell viability, integrity, and the levels of tight junction proteins of the intestine (see Examples 1 and 2). Further, the combination of the present invention is absorbed at the intestinal level without eliciting any irritation or cytotoxicity at this site. Then, the present combination can be administered orally. Moreover, the combination of the present invention preserves epithelial integrity and facilitate ion exchange across the intestinal barrier, indicating that it can traverse the cell monolayer without exerting a deleterious effect on the epithelium better than the reference product (see Example 2). The combination of the present invention demonstrates a notable capacity to reach a specific plasma concentration within four hours following administration, surpassing both the control and the reference product (p < 0.05) (see Example 2). Thus, the present combination enhances absorption during the physiological time of intestinal digestion and improves bioavailability leading to an improved management of venous disorders or other conditions where rapid intervention is required. Due to its oral bioavailability, the combination of the present invention is more convenient than other treatments requiring injections or more invasive administration.

The present combination of natural extracts is effectively absorbed and transported to the target site without adverse effects while preserving its biological activity. The combination of the invention is efficiently absorbed and distributed at the venous level, where it exerts its biological functions, ameliorating the condition of venous insufficiency by augmenting nitric oxide (NO) production (see Example 4).

The combination of the present invention stimulates cell viability, induces ATP production, and reduces ROS and pro-inflammatory cytokine production in venous endothelial cells to a greater extent than the reference product and without causing adverse effects (see Example 3).

The present combination is also able to modulate integrin, elastin and endothelin-1 levels. Specifically, the combination of the present invention restores transepithelial electrical resistance (TEER) levels to a higher level than the control, restoring levels altered by KCl damage. Similarly, elastin levels, used as a marker of vascular contraction/relaxation movements, were also improved after treatment with the combination of the present invention compared to KCl damaged sample (see Example 4). Moreover, endothelin-1 levels were also significantly improved after treatment with the combination of the present invention (see Example 4). The combination of the present invention also restores eNOS levels and consequently NO production, reversing the damaging trend of KCl (see Example 4). The combination of the present invention showed a greater effect in this context, preventing the decrease in eNOS and enhancing its expression more effectively than the reference product. Compared with the reference product, the combination of the present invention exhibited major efficacy in reducing MMP-9 and restoring VEGF, thereby supporting its use to counteract the processes leading to CVD and to restore vascular homeostasis during the disease state.

All these unexpected effects render the combination of the present invention particularly suitable for the treatment of venous diseases, in particular CVD or venous insufficiency.

It has been surprisingly found that a combination of diosmin, hesperidin, *Ruscus* extract, *Ananas* extract, and bromelain synergistically improves endothelium tone, integrity and response.

The composition of the present invention ameliorates the condition of venous insufficiency by stimulating cell viability, inducing ATP production, and reducing ROS and proinflammatory cytokine production in venous endothelial cells better than known products without causing adverse effects. Moreover, it reduces vascular permeability. Overall, the present composition has a protective effect against vascular degeneration and promotes vascular response.

Thus, one aspect of the invention relates to a composition comprising diosmin, hesperidin, *Ruscus* extract, *Ananas* extract and bromelain.

Another aspect of the present invention relates to a composition comprising diosmin, hesperidin, *Ruscus aculeatus* extract, *Ananas comosus* extract, and bromelain for use in the treatment and/or prevention of venous disease.

### Brief description of the figures

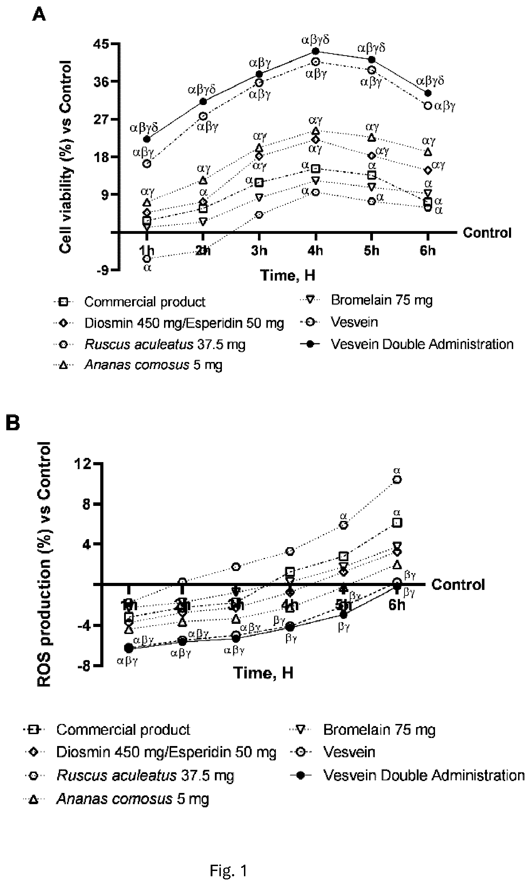

**Figure 1****.** Safety evaluation of Vesvein and individual agents on intestinal epithelium to exclude toxicity. In (A), the analysis of cell viability was done from 1h to 6h (H) using the MTT test; in (B), the ROS production was analysed from 1h to 6h (H) using Cytochrome C reduction. Control = not treated sample, Commercial product = film-coated tablet containing 500 mg of micronized purified flavonoid fraction consisting of 450 mg of diosmin and 50 mg of flavonoids expressed as hesperidin; diosmin/hesperidin = 450 mg + 50 mg (ratio 9:1); *Ruscus aculeatus* = 37.5 mg; *Ananas comosus =* 5 mg; bromelain = 75 mg; Vesvein = diosmin/hesperidin + *Ruscus aculeatus + Ananas comosus* + bromelain; Vesvein Double Administration = Vesvein combination administered 2 time for each point of treatment. Data are expressed as mean ± SD (%) of 5 independent experiments normalised to the control (corresponding to not treated sample) (0%) line. α p<0.0001 vs Control (line 0); β p<0.0001 vs single agents; γ p<0.0001 vs Commercial product; δ p<0.0001 vs Vesvein.

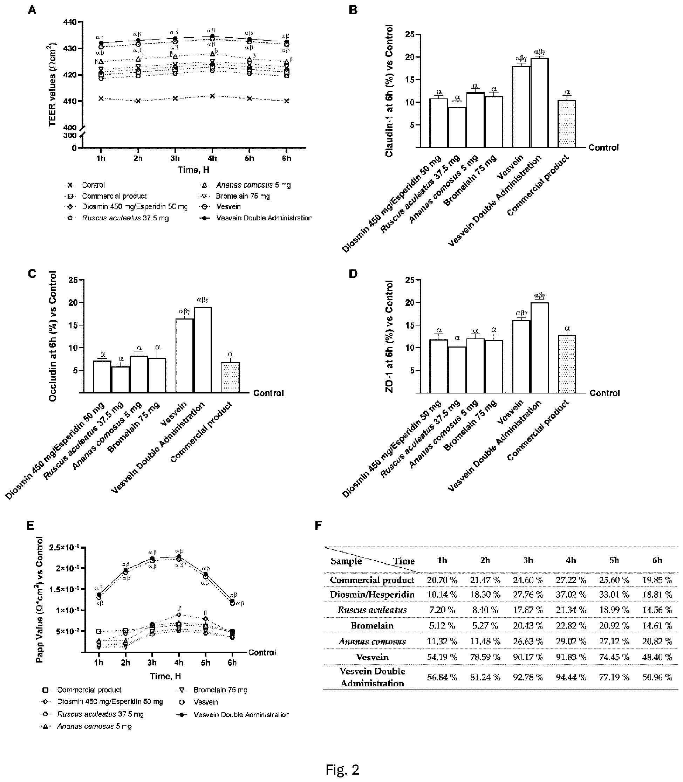

**Figure 2****.** Integrity and permeability analysis on a 3D model of intestinal barrier *in vitro.* In (A), TEER Value measurement using EVOM3 during the time (from 1h to 6h); from (B-D), the analysis of TJ measured by Enzyme-Linked Immunosorbent Assay (ELISA) test (Claudin-1, Occludin and ZO-1, respectively) at 6h; in (E), the Papp values during time (from 1h to 6h) obtained from the application of following formula: Papp = dQ/dt 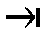 1/m0 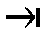 1/A 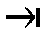 V Donor (1). In (F), data showing % permeability (from 1h to 6h) of samples across the intestinal barrier derived from Papp data
   Control = not treated sample, Commercial product = film-coated tablet containing 500 mg of micronized purified flavonoid fraction consisting of 450 mg of diosmin and 50 mg of flavonoids expressed as hesperidin; diosmin/hesperidin = 450 mg + 50 mg (ratio 90-10%); *Ruscus aculeatus* = 37.5 mg; *Ananas comosus =* 5 mg; bromelain = 75 mg; Vesvein = diosmin/hesperidin + *Ruscus aculeatus + Ananas comosus* + bromelain; Vesvein Double Administration = Vesvein combination administered 2 time for each point of treatment. In (A), data are expressed as means ± SD of five independent experiments. From (B-D), means ± SD were expressed by comparing data to the control (0% line) of five independent experiments performed in triplicates; in (E), the Papp values in which data < 0.2 × 10-6 cm/s mean very poor absorption with a bioavailability < 1%, data between 0.2 × 10-6 and 2 × 10-6 cm/s with bioavailability between 1 and 90%, and data > 2 × 10-6 cm/s mean very good absorption with a bioavailability over 90%. All substances were p<0.05 vs Control (line 0); α p<0.0001 vs Control (line 0); β p<0.0001 vs single agents; γ p<0.0001 vs Commercial product.

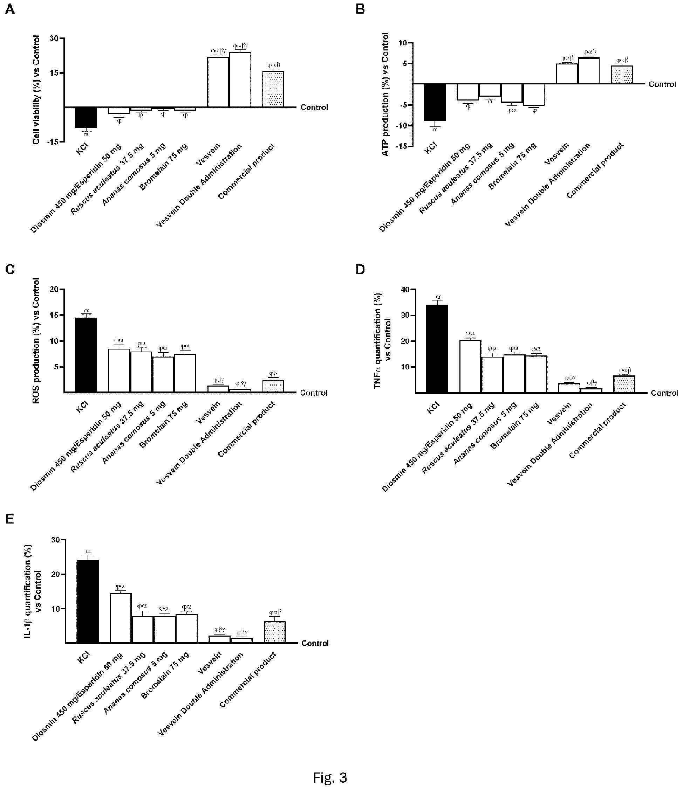

**Figure 3****.** Effect of individual components and combinations on the 3D vein model. In (A), analysis of cell viability by MTT test; in (B), analysis of ATP production by specific Kit; in (C), analysis of ROS production by Cytochrome C reduction; in (D), TNFα production analysis by ELISA Kit; and in (E), IL-1β production analysis by ELISA Kit. diosmin/hesperidin = 450 mg + 50 mg (ratio 90-10%); *Ruscus aculeatus* = 37.5 mg; *Ananas comosus =* 5 mg; bromelain = 75 mg; Vesvein = diosmin/hesperidin + *Ruscus aculeatus + Ananas comosus* + bromelain; Vesvein Double Administration = Vesvein combination administered 2 time for each point of treatment; Commercial product = film-coated tablet containing 500 mg of micronized purified flavonoid fraction consisting of 450 mg of diosmin and 50 mg of flavonoids expressed as hesperidin; KCl=96 mM ; Control = not treated sample. Data are expressed as mean ± SD (%) of 5 independent experiments normalised to the control (0%) line. α p<0.05 vs Control (line 0); β p<0.05 vs single agents; γ p<0.05 vs Commercial product; φ p<0.05 vs KCl.

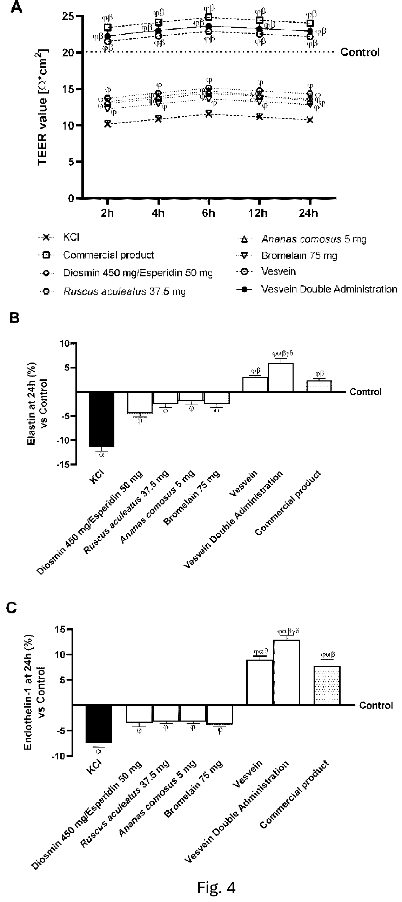

**Figure 4****.** Effect of individual components and combinations on the 3D vein model. In (A), TEER Value measurement using EVOM3 during the time (from 2h to 24h); in (B), quantification analysis of Elastin levels at 24 hours (h) by specific Kit; and in (C), quantification analysis of Endothelin-1 levels at 24 hours (h) by specific Kit. diosmin/hesperidin = 450 mg + 50 mg (ratio 90-10%); *Ruscus aculeatus* = 37.5 mg; *Ananas comosus =* 5 mg; bromelain = 75 mg; Vesvein = diosmin/hesperidin + *Ruscus aculeatus + Ananas comosus* + bromelain; Vesvein Double Administration = Vesvein combination administered 2 time for each point of treatment, Commercial product = film-coated tablet containing 500 mg of micronized purified flavonoid fraction consisting of 450 mg of diosmin and 50 mg of flavonoids expressed as hesperidin; KCl=96 mM; Control = not treated sample. Data are expressed as mean ± SD (%) of 5 independent experiments normalised to the control (0%) line. In (A), β p<0.05 vs single agents; φ p<0.05 vs KCl. From (B-C), α p<0.05 vs Control (line 0); β p<0.05 vs single agents; γ p<0.05 vs Commercial product; *δ* p<0.05 vs Vesvein; φ p<0.05 vs KCl.

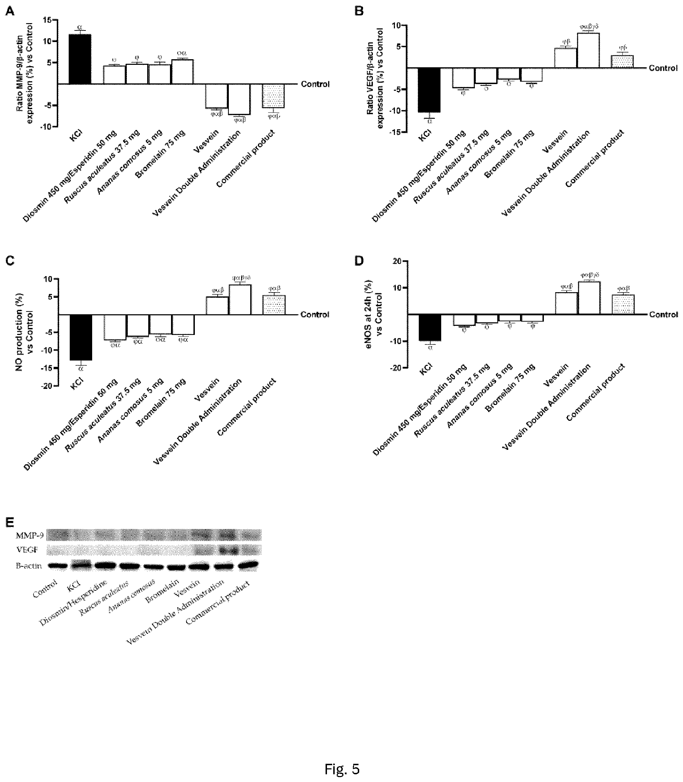

**Figure 5****.** Effect of individual components and combinations on the 3D vein model. In (A), MMP-9 expression by Western Blot analysis; in (B), VEGF expression by Western Blot analysis; in (C), quantification analysis of NO production by specific Kit; in (D), analysis of eNOS levels at 24 hours (h) by specific Kit; and in (E), an example of lane from Western Blot analysis for MMP-9, VEGF and B-actin. For panels A-E: diosmin/hesperidin = 450 mg + 50 mg (ratio 90-10%); *Ruscus aculeatus* = 37.5 mg; *Ananas comosus =* 5 mg; bromelain = 75 mg; Vesvein = diosmin/hesperidin + *Ruscus aculeatus + Ananas comosus* + bromelain; Vesvein Double Administration = Vesvein combination administered 2 time for each point of treatment, Commercial product = film-coated tablet containing 500 mg of micronized purified flavonoid fraction consisting of 450 mg of diosmin and 50 mg of flavonoids expressed as hesperidin; KCl=96 mM; Control = not treated sample. Data are expressed as mean ± SD (%) of 5 independent experiments normalised to the control (0%) line. In (A), α p<0.05 vs Control (line 0), β p<0.05 vs single agents; φ p<0.05 vs KCl, From (B-D), α p<0.05 vs Control (line 0); β p<0.05 vs single agents; γ p<0.05 vs Commercial product; *δ* p<0.05 vs Vesvein; φ p<0.05 vs KCl.

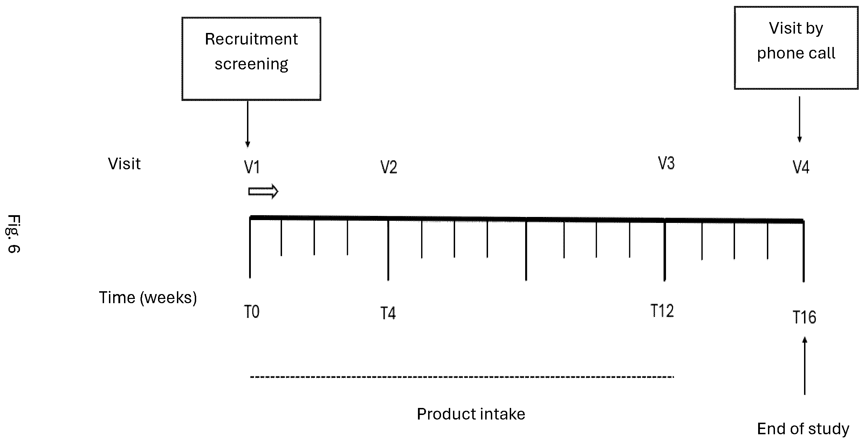

**Figure 6****.** Clinical study scheme. The study consisted of a recruitment screening followed by 12 weeks of product (=Vesvein, i.e. = diosmin/hesperidin + *Ruscus aculeatus + Ananas comosus* + bromelain) intake. The study ended after 16 weeks from it start with a visit by phone call. Scheduled visits (V=visit) at the corresponding weeks (T= time expressed in weeks) from the start of the study are also shown.

### Detailed description of the invention

### Definitions

The term "diosmin" as used herein refers to a naturally occurring flavonoid glycoside that can be isolated from various plant sources or derived from the flavonoid hesperidin. It is chemically described as 5-hydroxy-2-(3-hydroxy-4-methoxyphenyl)-7-[(2S,3R,4S,5S,6R)-3,4,5-trihydroxy-6-[[(2*R*,3*R*,4*R*,5*R*,6*S*)-3,4,5-trihydroxy-6-methyloxan-2-yl]oxymethyl]oxan-2-yl]oxychromen-4-one. Diosmin functions primarily as a venotonic agent by decreasing venous distensibility and fortifying vein walls. As a result, the heart receives more blood return, and the venous tone is improved.

Its ability to block the production of prostaglandin and histamine efficiently reduces inflammation in the walls of vessels. Furthermore, it is essential for microcirculation because it maintains the stability of capillary walls and stops fluid from seeping into neighbouring tissues. Its all-encompassing influence on vascular health is further shown by the improvement of lymphatic drainage and antioxidant activity. Diosmin also supports smooth muscle contraction, which helps to sustain ideal vascular function [53].

Pharmacokinetic investigations have shown that diosmin is rapidly transformed by intestinal flora to its aglycone form, diosmetin. Diosmetin is absorbed and rapidly distributed throughout the body with a plasma half-life of 26-43 hours. Diosmetin is degraded to phenolic acids or their glycine-conjugated derivatives and eliminated through the urine. Diosmin or diosmetin not absorbed is eliminated in the feces. A study demonstrated that diosmin can prolong the duration of norepinephrine-induced venous contraction in isolated human normal and varicose veins, likely due to the fact that diosmin enhances the physiological effects of norepinephrine on the venous wall. In addition to its effect on venous tone, diosmin was found in animal models to have anti-inflammatory effects through reduction of PGE2 and thromboxane A2 synthesis, which contribute to diosmin beneficial effects on venous disorders. Moreover, diosmin treatment was found to significantly lower serum levels of the proinflammatory cytokines (TNF-a, IL-6, and IL-1b) in a mouse model.

Diosmin mechanisms of action include the improvement of venous tone, increased lymphatic drainage, protection of capillary bed microcirculation, inhibition of inflammatory reactions, and reduced capillary permeability.

Diosmin also demonstrated multiple desirable properties in several clinical studies. A study compared the effects of micronized and non-micronized diosmin on patients with CVD of the lower limbs. Both formulations were found to significantly improve different disease signs and symptoms compared to baseline values. Micronized fraction showed significantly stronger effects than the non-micronized one. For example, micronised diosmin improved heaviness and leg pain more than the non-micronised form. Diosmin also showed antioxidant effects in a clinical study measuring the levels of isoprostanes, reliable markers of oxidative stress, in CVD patients treated with 600 mg diosmin twice daily for 3 months. The results of this study showed that diosmin treatment significantly lowered blood isoprostane levels, indicating its antioxidant effect.

The term "hesperidin" as used herein refers to a flavanone glycoside found in citrus fruits. It is chemically described as (2*S*)-5-hydroxy-2-(3-hydroxy-4-methoxyphenyl)-7-[(2*S*,3*R*,4*S*,5*S*5,6*R*)-3,4,5-trihydroxy-6-[[(2*R*,3*R*,4*R*,5*R*,6*S*)-3,4,5-trihydroxy-6-methyloxan-2-yl]oxymethyl]oxan-2-yl]oxy-2,3-dihydrochromen-4-one. Hesperidin has antioxidant activity, which is related to the aglycone structure, particularly, to the presence of a hydroxyl group in position C30 and a methoxyl group in position C40 in ring B. The mechanism underlying this activity relies on its capacity to scavenge free radicals or reactive oxygen ions, such as superoxide and hydrogen peroxide, which usually results in a lower activity of detoxifying enzymes. This flavanone has proven antioxidant and anti-inflammatory activities, which potentiated hesperidin investigation as an anticancer, antiviral, anti-obesity, anti-aging, cardio- and neuroprotective agent [54].

Diosmin and hesperidin may be present in a balanced blend of bioflavonoids, present in micronized form, which allows improving the intestinal absorption of drugs ensures high and rapid intestinal absorption.

Diosmin and hesperidin are flavonoids derived from botanical sources. Flavonoids are a class of phytochemicals produced by different plants. Chemically, flavonoids are divided into several classes including flavones, flavanones, and flavonols. Flavonoids are known for their desirable pharmacological properties such as antioxidant and anti-inflammatory effects. Micronized purified flavonoid fraction (MPFF) is a phlebotonic, with evidence supporting its use in relieving symptoms/signs of CVD and improving quality of life. International guidelines recommend venoactive drugs (VADs), among other conservative treatments, to improve symptoms and signs of CVD.

Diosmin is a naturally occurring flavonoid glycoside that can be isolated from various plant sources or derived from the flavonoid hesperidin. Diosmin differs molecularly from hesperidin by the presence of a double bond between two carbon atoms in diosmin central carbon ring. Diosmin can be manufactured by extracting hesperidin from citrus rinds, followed by conversion of hesperidin to diosmin. Diosmin is considered to be a vascular-protecting agent used in chronic venous disease and haemorrhoids. As a flavonoid, diosmin exhibits anti-inflammatory, free-radical scavenging properties. Diosmin has been used for more than 30 years as a phlebotonic and vascular-protecting agent.

Hesperidin is a prominent flavanone found in citrus fruits that has a broad range of biological effects, including anti-inflammatory and antioxidant capabilities. Hesperidin is the food-bound form of hesperetin and is one of the two molecules which were previously erroneously named "Vitamin P". Numerous *in vivo* and *in vitro* studies have been performed to evaluate effects of hesperidin, showing its potential benefits in a variety of conditions, including cardiovascular diseases.

Hesperidin and hesperetin enhance cellular antioxidant defence by eliminating free radicals and ROS (reactive oxygen species). Specifically, hesperidin and hesperetin effectively neutralize ROS, including superoxide anions, hydroxyl radicals, peroxynitrite and NO radical. Oxidative stress frequently elicits an accompanying inflammatory response. Hesperidin and hesperetin inhibit various inflammatory pathways; they inhibit inflammatory body (inflammasome) activation and downregulate IL-1β and TNFα levels as well as the inflammatory mediators IL-6 and carbon monoxide (CO).

Hesperidin has a limited bioavailability due to low water solubility, so that only a small quantity of hesperidin molecules is released into the aqueous environment of the gastrointestinal tract.

Hesperidin is poorly absorbed and rapidly eliminated; however, it has a reasonable half-life of 6 h, probably due to the prolonged absorption phase, as shown by a long Tmax. Due to the variety of pharmacological activities in the human body, hesperidin is one of the most interesting and promising bioflavonoids. The biological and pharmacological properties of hesperidin have been extensively studied *in vitro* and in animal models to reveal its antioxidant, anti-inflammatory effects. Protective effects of hesperidin on the vascular system are expressed in increasing in the expression of antioxidative enzymes. Together with the flavone diosmin, it has been shown to decrease capillary fragility in venous circulation disorders.

The term *"Ruscus* extract" as used herein refers to an extract of any *Ruscus* species, for instance *Ruscus aculeatus* (butcher's broom), *Ruscus colchicus, Ruscus hypoglossum, Ruscus hypophyllum, Ruscus Hyrcanus, Ruscus microglossus, Ruscus murtae* or *Ruscus streptophyllus.* Preferred *Ruscus* species is *Ruscus aculeatus.* Any part of *Ruscus* may be used to produce the extract, in particular leaves and roots, preferably roots.

The *Ruscus* extract, in particular *Ruscus aculeatus* extract, encompasses active compounds such as ruscogenin, neoruscogenin, and various other saponins. Preferably the *Ruscus* extract contains or comprises at least from about 5 % (w/w) to about 15 % (w/w) of ruscogenin (w/w), preferably from about 5 % (w/w) to about 10 % (w/w) of ruscogenin, more preferably about 10 % (w/w) of ruscogenin ((w/w), weight/weight of the total weight of the extract). *Ruscus* extract, in particular *Ruscus aculeatus* extract has vasoconstrictive activity, which is attributed to the agonistic interaction with α-1 and α-2 adrenergic receptors located in the vascular wall, alongside the facilitation of norepinephrine release from adrenergic nerve terminals [20]. Furthermore, *Ruscus* extract, in particular *Ruscus aculeatus* extract, significantly affects venous structures, lymphatic vessels, and capillary networks. Specifically, a variety of mechanisms have been proposed to elucidate the protective effects of *Ruscus* extract on microcirculation, which include vasoconstriction of vascular structures leading to mitigated venous hypertension and localised protective effects associated with the safeguarding of endothelial cells and anti-inflammatory characteristics. A noteworthy advantage of using *Ruscus* extract, in particular *Ruscus aculeatus* extract is its ability to inhibit the vascular wall's augmented permeability, a process mediated by histamine [21]. In general *Ruscus,* in particular *Ruscus aculeatus,* is a traditional herbal medicinal product to relieve symptoms of discomfort and heaviness of legs related to minor venous circulatory disturbances. *Ruscus aculeatus* L. (Asparagaceae), also known as butcher's broom, is a low evergreen shrub and features in many dietary supplement patents. Its root is used as a phytotherapeutic product. The bioactive compounds identified in the *Ruscus aculeatus* L. extracts include saponins, flavonoids, sterols, tyramine, coumarin, triterpenes, lignoceric acid, glycolic acid, and benzofurans. The beneficial effects of *Ruscus aculeatus* L. on microcirculation include its venotonic activities. Phlebotonic properties of *Ruscus* extracts are well known despite the mechanism of its action not being fully explained. *Ruscus aculeatus* seems to trigger vasoconstriction by activating both α1 and α2 adrenergic receptors as well as by directly acting on venous smooth muscle cells. In addition, *Ruscus* extracts exhibit inhibitory effects on biologically active substances which increase vascular permeability such as bradykinin, leukotriene B4 and histamine. Sterolic saponins contained in *Ruscus aculeatus* extracts, particularly the best-studied ruscogenin, are ascribed with anti-inflammatory, elastase-inhibiting, as well as vessel permeability-reducing effects. The mechanism of antiedemic activity stems from the reduction of capillary permeability for proteins and inhibition of increased capillary filtration.

*Ruscus* extract, in particular *Ruscus aculeatus* extract, shows many effects such as antioxidant activity (improvement of the total antioxidant capacity, scavenger of free radicals including hydroxyl and superoxide radicals, chelation of transition metal ions, prevention against oxidation of cellular macromolecules such as proteins, lipids, carbohydrates and DNA and inhibition of lipid peroxidation), anti-inflammatory activity (inhibition of neutrophil adhesion to endothelial cells, inhibition of leukocyte migration, inhibition of expression of VACAM-1 and ICAM-1, inhibition of induction of proinflammatory cytokines, mainly TNF-a, inhibition of activation of the NFkB pathway), vascular action (improvement of blood flow, inhibition of activity of elastase (enzyme responsible for damage to elastin fibres), increase of norepinephrine release, reduction or blood retention, decrease of the capillary filtration rate, stimulation of the release of noradrenaline to increase vascular tone and constrict blood vessels, reduction of leg swelling and oedema), and other actions (increasing HDL level, decreasing in HbAlc level, decreasing total cholesterol level, reduction of blood glucose, intensification of lymphatic drainage, intensification of healing process in ulcers). Moreover, *Ruscus aculeatus* has been shown to reduce vascular permeability. *Ruscus* extract led to a significant decrease in the venous diameter of deep veins and at the same time to an increase in flow parameters. *Ruscus* extract was also shown to decrease capillary filtration rate in healthy volunteers and patients with CVD. Additionally, it was shown that the increase in venous tone and the reduction in capillary filtration resulted in an increase in lymph flow in patients with oedema.

The term *"Ananas* extract" as used herein refers to an extract of pineapple stem. The *Ananas* may be *Ananas comosus L. Merril or Ananas macrodontes.*

*Ananas* extract, in particular *Ananas comosus* extract contains various amino acids, including tyrosine and tryptophan, bromelain and tacorin, together with saponin, flavonoids, phytosterol, carbohydrates, alkaloids, cardiac glycosides, amino acids, and proteins.

The *Ananas* extract, in particular *Ananas comosus* extract, has many pharmacological activities, such as: antimicrobial activity, antibacterial activity, anticancer activity, antioxidant activity, anti-inflammatory activity, antidepressant activity, hepatoprotective activity, anti-rheumatic activity, anti-ageing activity, anti-acetylcholinesterase activity, cardioprotective property, anti-coagulant activity, anthelmintic activity, anti-diabetic activity and wound healing [55].

The *Ananas* extract that has a bromelain enzymatic activity of about 50 GDU/g of bromelain to about 2500 GDU/g of bromelain, preferably at least 100 GDU/g of bromelain, preferably at least 250 GDU/g of bromelain.

The term "Bromelain" as used herein refers to a group of proteolytic enzymes, primarily found in the stem and fruit of pineapple (*Ananas*), that breaks down proteins and have several properties. In the present invention bromelain can be produced from any *Ananas* species, in particular *Ananas comosus* (specifically *Ananas comosus* L. Merril) or *Ananas macrodontes.* Both stem and fruit bromelain are isolated as glycosylated, monomeric single proteins with an isoelectric point (pI) value of 9.55 and optimum pH range of 6-7. The proteins also contain seven cysteine residues and hence a total of three disulfide bonds. As such, they can also be further classified as cysteine proteinases, belonging to the common group of sulfhydryl proteolytic enzymes. Bromelain is also considered a thiol endopeptidase (internal clearing enzyme), with the activity of all sulfhydryl endopeptidases depending upon the thiol group (SH) of a cysteine residue. Stem bromelain has a stable secondary structure and shows activity in the pH range 7-10, with its activity being irreversibly lost above pH 10 [56]. Bromelain reduces inflammation and oedema by modulating proinflammatory cytokines and improving microcirculation [22]. Furthermore, Bromelain has a crucial role as a fibrinolytic, anticancer, antibiotic, anticoagulant, and antithrombotic agent. In addition, it may have beneficial analgesic-type properties that can help to heal and minimize post-surgical pain and swelling. Furthermore, bromelain improves recovery after tissue damage and boost drug absorption, particularly for antibiotics [56]. Bromelain helps drain body fluids.

Bromelain belongs to the Bromeliaceae family and is a protein-digesting enzyme derived from the pineapple fruit or, more commonly, stem. The pineapple plant possesses therapeutic characteristics: it contains several proteolytic enzymes, such as comosain and ananain, but bromelain is the most studied. Bromelain is a combination of several thiol endopeptidases and various protease inhibitors, glucosidase, cellulase, phosphatase, peroxidase, and escharase. Bromelain was discovered in 1891, but it took a long time to thoroughly isolate, purify, and describe it. As proteolytic enzyme (protease), bromelain catalyses the breakdown of proteins into amino acids. It is prepared from the juice of cooled pineapple through ultrafiltration, centrifugation, and lyophilization, which results in the production of a yellow powder and the extraction and purification process has several steps. *Ananas comosus* is a tropical fruit which contains considerable amounts of bioactive compounds, dietary fibre, minerals, and nutrients. In addition, it has been proven to have various health benefits including anti-inflammatory and antioxidant activity. The action of bromelain is primarily attributed to its ability to break down proteins. Bromelain cleaves peptide bonds in proteins, causing them to break into smaller peptides and amino acids. This process can help in reducing inflammation by breaking down inflammatory proteins such as cytokines and chemokines. In addition to its proteolytic activity, bromelain has also been shown to have other molecular targets. For instance, bromelain has been found to inhibit the activity of cyclooxygenase (COX) enzymes, which are involved in the production of inflammatory prostaglandins. The anti-inflammatory activity of *Ananas comosus* was examined in a carrageenan-induced inflammatory rat model. *Ananas comosus* showed significant inhibition of protein denaturation and proteinase activity and also controlled secretion of inflammatory cytokines and prostaglandins, as well as the generation of reactive oxygen species by activated macrophages. *Ananas comosus* also significantly decreased carrageenan-induced acute paw oedema. Molecular mechanisms by which bromelain exerts its effects on the cardiovascular system are complex and multifactorial. It appears to act through several pathways, including inhibition of platelet aggregation, fibrinolysis, anti-inflammatory effects, and activation of signalling pathways involved in cellular processes. Bromelain's fibrinolytic effect is due to the presence of enzymes in bromelain that can convert plasminogen, an inactive precursor, to plasmin, an active enzyme that can break down fibrin. Bromelain fibrinolytic capacity allows reducing plasma levels of fibrinogen and bradykinin, resulting in less oedema and inflammation, thus allowing greater reabsorption of fluids at the interstitial level. Bromelain's proteolytic activity facilitates the breakdown of proteins involved in oedema formation and swelling. By degrading extracellular matrix proteins and reducing the accumulation of fluid in tissues, bromelain may help alleviate inflammation-associated oedema. In animal models, *Ananas comosus* demonstrated to reduce exudates formation, probably via inhibition of pro-inflammatory mediators. *In vitro* and *in vivo* studies have shown that bromelain is a fibrinolytic agent. It stimulates the increased conversion of plasminogen to plasmin, resulting in increased degradation of fibrin. Furthermore, it has been shown that bromelain causes a decrease in platelet aggregation. These results were confirmed in later studies conducted both *in vitro* and *in vivo,* where bromelain was shown to cause platelet inhibition in a dose-dependent manner. It has also been shown that bromelain initiates thrombus destruction, as well as reduces platelet clumping and lowers blood viscosity. Combined with diosmin, it was shown that both compounds slightly reduced the internal viscosity of the red blood cells (RBCs), had a stabilizing effect on the cell membrane and improved the rheological properties of the RBCs. As anticipated by its several pharmacodynamic properties, bromelain has various therapeutic properties, including circulation improvement. It is used to modulate blood coagulation, inflammation and oedema among many other conditions. In particular, it is regarded as a potent anti-oedematous and anti-inflammatory agent that works on a variety of targets that respond to inflammation. It is also used to combat thrombophlebitis. Studies have shown the potential of bromelain for the treatment of circulatory and venous diseases. Orally administered bromelain was shown to reduce the oedema in traumatically induced hind leg oedema in rats. *Ananas comosus* has been shown to have various health benefits including anti-inflammatory and antioxidant activity, with potential therapeutic uses. In addition, it contains enzymes related to damage tissue repairing and wound healing.

The term "GDU" or "Gelatin Digestion Unit" refers to the enzymatic activity of bromelain. One unit hydrolyses 1.0 mg of amino nitrogen from gelatin in 20 minutes at pH 4.5 at 45°C. The term "GDU/g" refers to the enzymatic activity per gram of bromelain. Bromelain enzymatic activity measurement is known in the art and any method known in the art can be used. In particular, the method using sodium caseinate as substrate, as described by Fissore et al. (2023) [60].

The term "Venous Disease" as used herein refers to chronic venous insufficiency (CVI) or chronic venous disease (CVD) and complications thereof. Examples of chronic venous disease comprises chronic deep vein thrombosis (DVT), induced venous hypertension, phlegmasia (acute DVT) alba dolens, phlegmasia cerulea dolens, effort thrombosis (upper arm) ("Paget Shroeder disease"), chronic mesenteric venous disease, portal venous hypertension, or DVT induced pelvic congestion. Examples of complications or diseases of the veins (due to CVI or CVD) are to swelling and inflammation of veins in the rectum, anus and vulva; venous hypertension; increased permeability; oedema; capillary damage; skin changes; venous leg ulcers; swelling ankles; heavy legs; varicose veins; swelling leg; ulcers; vein thrombosis; phlebitis; thrombo-phlebitis; pulmonary embolus or haemorrhoids.

Patients suffering from Chronic venous disease can be classified using the Clinical Etiology Anatomy Pathophysiology (CEAP) classification, an internationally accepted standard for describing patients with chronic venous disorders [61]. The C designation include the following: C0 - No visible or palpable signs of venous disease; C1 - Telangiectasias or reticular veins; C2 - Varicose veins; C2r - Recurrent varicose veins; C3 - Edema; C4 - Changes in skin and subcutaneous tissue secondary to CVD; C4a - Pigmentation or eczema; C4b - Lipodermatosclerosis or atrophie blanche; C4c - Corona phlebectatica; C5 - Healed ulcer; C6 - Active venous ulceration and C6r - Recurrent venous ulceration.

Each clinical class sub characterized by a subscript indicating the presence (symptomatic, s) or absence (asymptomatic, a) of symptoms attributable to venous disease. The composition of the invention may be used for the treatment and/or prevention of any the above class of CVD.

The term "treat" or "treating" or "treatment" as used herein includes alleviating, ameliorating, giving relief to symptoms associated to venous disease.

The term "prevent" or "preventing" or "prevention" as used herein designates stopping or at least decreasing the probability of occurrence of venous disease.

The term "improve endothelium tone, integrity and response" may encompass:
- Improve endothelium repair: such as (but not limited to) recovery of the integrity of the endothelium, such as repair of endothelium, reduction of permeability upon inflammatory challenge
- Improved endothelium structure: such as (but not limited to) integrity of the vascular endothelial barrier, tight junction structure, and endothelium lining integrity.
- Improved endothelium response: such as (but not limited to) endothelium-dependent relaxation response, endothelium-dependent vasoconstriction response, inflammatory response of the vascular endothelium.

The term "increased cell viability" as used herein refers to an *in vitro* increase of cell viability in particular to an increase from at least 2-fold, preferably at least 4-fold vs. KCl treated sample.

The term "increased anti-oxidative effect" as used herein refers to *in vitro* reduction of ROS production. in particular an increased anti-oxidative effect corresponds to a reduction of ROS production ranging from 70% to 100% vs. KCl treated sample.

The term "increased ATP production" as used herein refers to *in vitro* restored ATP production. In particular it corresponds to an increase of ATP level ranging by 1.5 to 3 folds or more vs. KCl treated sample.

The term "decreased inflammatory response" as used herein refers to the reduction of proinflammatory cytokines, such as TNFα and IL-1β, in particular to from 70% to 100% vs. KCl treated sample.

The term "improved TEER measurement" as used herein refers to the improvement of barrier integrity. In particular an improved TEER measurement corresponds to an increase of TEER measurement ranging from 1.5 to 3 folds or more vs. KCl treated sample.

The term "improved elastin levels" as used herein refers to the regulatory effect on vessel contraction and relaxation movements. In particular, an improved elastin level corresponds to an increase of elastin level ranging from 1 to 2 folds or more vs. KCl treated sample.

The term "improved endothelin-1 levels" as used herein refers to the ability of enhancing neovascularization. An improved endothelin-1 level corresponds to an increase of endothelin-1 level ranging from 1 to 2 folds or more vs. KCl treated sample.

The term "increasing the level of VEGF" or "increasing the level of vascular endothelial growth factor" as used herein refers to the enhancement of angiogenesis and inflammatory response. In particular, an increased VEGF level corresponds to an increase of VEGF level ranging from 40% to 70% or more vs. KCl treated sample.

The term "reduced expression of MMP-9" or "reduced expression of Matrix Metalloproteinase-9" as used herein refers to the reduction of venous wall dilatation. In particular to a reduced expression of MMP-9 level corresponds to a reduction of MMP-9 expression ranging from 2 to 3 folds or more vs. KCl treated sample.

The term "increased eNOS levels" as used herein refers to an *in vitro* increase of eNOS levels. In particular an increase from 1-fold to 2-fold or more vs. the single agents.

The term "increased NO levels" as used herein refers to an *in vitro* increase of NO levels. In particular from 1-fold to 2-fold or more vs. the single agents.

The term "CIVIQ-20" or "Chronic Venous Insufficiency quality of life Questionnaire" as used herein refers to a 20-item questionnaire administered to chronic venous disease (CVD) patients to assess quality-of-life providing a global index and an outline of 4 quality-of-life dimensions: "pain" (4 items), "physical" (4 items), "psychological" (9 items), and "social" (3 items) in patients with chronic venous disease. Items on the CIVIQ-20 scale are scored from 1 to 5. A low score corresponds to greater patient comfort.

The terms "GIS" or "Global Index Score" refer to a standardized score deriving from CIVIQ-20 questionnaire. Improvement in quality of life between two study times is represented by a decrease in score.

The term "100-point VAS" as used herein refers to a Visual Analogue Scale (VAS) used to record patients' pain progression and compare pain severity between patients with similar conditions. A higher score indicates greater pain intensity.

The terms "Venous Disability Score" or "VDS" as used herein refers to a method which evaluates the effect of venous disease by quantifying the level of work-based disability. It is scored on a scale of 0 (asymptomatic) to 3 (unable to work an 8-hour day, even with support), based on the ability to work an 8-hour day with or without provisions for external support. The total score represents the degree of disability attributable to venous disease.

The term "CEAP classification" as used herein refers to a grading system to indicate the severity of the varicose vein problem. The CEAP classification ranges from C0 (absolutely no venous disease that can be seen or felt in the legs) to C6 (an open and active venous leg ulcer).

The invention will now be described in further details. It is noted that the various aspects, features, examples and embodiments described in the present application may be compatible and/or combined together.

It has now been found that a composition comprising diosmin, hesperidin, *Ruscus aculeatus* extract, *Ananas comosus* extract and bromelain, is endowed with a surprisingly synergistic effect particularly advantageous for the prevention and/or treatment of venous disease.

In particular, the compositions of the invention improve endothelium tone, integrity and response. The compositions of the invention also increase cell viability and increases antioxidative effect as measured by *in vitro* reduction of ROS production. The compositions of the invention increase ATP production and decrease inflammatory response, for instance as shown by a reduction of pro-inflammatory cytokines, such as TNFα and IL-1β. The compositions of the invention improve barrier integrity as measured by TEER measurement and improve elastin levels, having a positive effect on vessel contraction and relaxation movements. The compositions of the invention further improved endothelin-1 levels as a marker of enhanced neovascularization and increase the level of VEGF, displaying enhancement of angiogenesis and decreasing inflammatory response. The compositions of the invention also reduce expression of MMP-9 thereby reducing venous wall dilatation and increase eNOS or NO levels. The compositions of the invention improved the quality of life of patient affected by venous disease, for instance as measured by "CIVIQ-20" or "Chronic Venous Insufficiency quality of life Questionnaire" or by "GIS" or "Global Index Score" or by "100-point VAS". The compositions of the present invention improve the "Venous Disability Score" or "VDS".

It is therefore an object of the present invention a composition comprising diosmin, hesperidin, bromelain, *Ruscus* extract and *Ananas* extract.

Preferably diosmin is in an amount from 27% to 90% (w/w) of the total weight of the composition.

In a preferred embodiment hesperidin is in an amount from 3% to 10% (w/w) of the total weight of the composition.

In a further preferred embodiment bromelain is in an amount from 4% to 10% (w/w) of the total weight of the composition.

Preferably the bromelain has an enzyme activity of at least 2500 GDU/g.

In a preferred embodiment the *Ruscus* extract is in an amount from 1% to 6% (w/w) of the total weight of the composition. Preferably the *Ruscus* extract comprises from 5% to 15% of ruscogenin, preferably the *Ruscus* extract comprises 10% of ruscogenin.

In a preferred embodiment the *Ananas* extract is in an amount from 0.1% to 2% (w/w) of the total weight of the composition.

Preferably the *Ananas* extract comprises bromelain having an enzymatic activity of between 100 to 500 GDU/g, preferably the *Ananas* extract comprises bromelain having an enzymatic activity of 250 GDU/g.

In a preferred embodiment the composition comprises about 44% (w/w) of diosmin, about 5% (w/w) of hesperidin, about 4% (w/w) of *Ruscus* extract, about 0.5% (w/w) of *Ananas* extract, and about 7.5% (w/w) bromelain of the total weight of the composition.

In a preferred embodiment the composition is for use in the treatment and/or prevention of a venous disease or a venous insufficiency. Preferably the venous disease or the venous insufficiency is chronic venous disease or chronic venous insufficiency. Still preferably the chronic venous disease is varicose veins.

In a preferred embodiment the composition is for use in the treatment and/or prevention of venous ulcers, thrombus formation, painful oedema, swelling, and distal venous hypertension. Preferably, the composition of the present invention comprises diosmin from 360 to 540 mg, hesperidin from 40 to 60 mg, *Ruscus aculeatus* extract from 30 to 45 mg, *Ananas comosus* extract from 1 to 10 mg and bromelain from 65 to 85 mg, wherein the final weight of the composition is about 1000 g.

More preferably, the composition of the present invention comprises diosmin 450 mg, hesperidin 50 mg, *Ruscus aculeatus* extract 37.5 mg, *Ananas comosus* extract 5 mg, and bromelain 75 mg, wherein the final weight of the composition is 1000.00 g.

In an aspect, the composition of the present invention comprises diosmin and hesperidin in a ratio of 15:1 to 1:15, preferably 9:1.

In an aspect, the composition of the present invention comprises about 44% (w/w) of diosmin, about 5% (w/w) of hesperidin, about 4% (w/w) of *Ruscus aculeatus* extract, about 0.5% (w/w) of *Ananas comosus* extract, and about 7.5% (w/w) bromelain referred to the weight of the final composition.

In an aspect, the composition of the present invention comprises diosmin in an amount from 27% to 90% (w/w) and hesperidin in an amount from 3% to 10% (w/w) referred to the weight of the final composition.

In an aspect, the composition of the present invention comprises *Ruscus aculeatus* extract in an amount from 1% to 6% (w/w) referred to the weight of the final composition.

In an aspect, *Ruscus aculeatus* extract of the composition of the present invention comprises from 5% to 15% of ruscogenin, preferably 10%.

In an aspect, the composition of the present invention comprises *Ananas comosus* extract in an amount from 0.1% to 2% (w/w) referred to the weight of the final composition. Preferably, the *Ananas comosus* extract comprises 100 to 500 GDU/g of bromelain, more preferably the *Ananas comosus* extract comprises 250 GDU/g of bromelain. In an aspect, the bromelain contained in the *Ananas comosus* extract has an enzymatic activity of 1.25 GDU.

In an aspect, the composition of the present invention comprises bromelain in an amount from 4% to 10% (w/w) referred to the weight of the final composition, preferably said bromelain is 2500 GDU/g. Preferably, bromelain has an enzymatic activity of 187.5 GDU.

The composition mentioned above may further comprise other active ingredients useful for treating diseases of the vein, such as co-enzymes, mineral substances, antioxidants, vitamins, probiotics, anticlotting agents and agents useful for treating diseases of the veins.

The tablet may further comprise commonly used excipients such as at least one bulking agent (such as microcrystalline cellulose), at least one stabilizer (such as hydroxy-propylmethylcellulose and/or cross-linked sodium carboxy methyl cellulose), at least one anticaking agent (such as silicon dioxide and/or magnesium salts of fatty acids). Further the tablet may or not be coated. The tablet may comprise a film coating comprising at least one glazing agent (such as hydroxy-propylcellulose and/or polyethylene glycol), at least one coloring agent (such as iron oxide) and optionally at least one sweetener (such as steviol glycosides from Stevia).

It is a further object of the invention a food supplement comprising the composition of the invention as described above and optionally further comprising one or more excipients or diluents.

The food supplement may further comprise other active ingredients useful for treating diseases of the vein, such as co-enzymes, mineral substances, antioxidants, vitamins, probiotics, anticlotting agents and agents useful for treating diseases of the veins.

The composition or the food supplement of the present invention can be administered orally, parenterally, intravenously, topically and/or transdermally, in any suitable form, preferably orally or topically. The oral administration is preferred.

In an aspect the composition or the food supplement is formulated as tablet, powder, pellet, granule, hard gelatin capsule comprising multiple beads, or soft gelatin capsule containing a lipid-based vehicle. Liquid dosage forms such as suspensions may be used as well. Preferably, the composition or the food supplement is in form of tablet. Even more preferably, the composition or the food supplement is in form of multilayer tablets, preferably double-layer tablets, with differentiated release of active ingredients.

The present composition may be specifically formulated in double-layer tablet for the differentiated release of *Ruscus* and bromelain. Double-layer tablets provide many advantages over conventional monolithic tablets. Through the process of multi-layer compression, different release profiles can be achieved simultaneously in a fixed-dose combination.

In an aspect, the composition or the food supplement according to the invention is administered one, two, three or four times in a day, preferably one or two times in a day.

It is a further object of the present invention the composition or the food supplement for use in the treatment and/or prevention of venous disease, preferably chronic venous insufficiency, chronic venous disease, complications or disease thereof.

In an aspect, said chronic venous disease comprises chronic deep vein thrombosis (DVT), induced venous hypertension, phlegmasia (acute DVT) alba dolens, phlegmasia cerulea dolens, effort thrombosis (upper arm) ("Paget Shroeder disease"), chronic mesenteric venous disease, portal venous hypertension, or DVT induced pelvic congestion.

In an aspect, said complications or disease due to chronic venous insufficiency or chronic venous disease are selected from the group comprising: swelling and inflammation of veins in the rectum, anus and vulva; venous hypertension; increased permeability; oedema; capillary damage; skin changes; venous leg ulcers; swelling ankles; heavy legs; varicose veins; swelling leg; ulcers; vein thrombosis; phlebitis; thrombo-phlebitis; pulmonary embolus or hemorrhoids. Preferably, the venous disease is chronic venous disease. More preferably, the composition of the present invention is useful for the treatment and/or prevention of varicose veins.

In an aspect, the composition or the food supplement according to the invention useful in the treatment and/or prevention of venous disease, is administered one, two, three or four times in a day, preferably one or two times in a day.

The precise effective dose for a human subject will depend upon the severity of the disease state, general health of the subject, age, weight, and gender of the subject, diet, time and frequency of administration, drug combination(s), reaction sensitivities, and tolerance/response to therapy. This amount can be determined by routine experimentation and is within the judgement of the clinician.

It is a further object of the present invention a nutritional composition comprising the composition of the invention. Preferably the nutritional composition is used for the prevention and/or treatment of chronic venous insufficiency, chronic venous disease and complications thereof.

All patent and non-patent references cited in the present application, are hereby incorporated by reference in their entirety.

The invention will now be described in further details in the following non-limiting examples.

### Examples

### Material and methods

### Agents and Compositions

### Commercial product (reference)

Film-coated tablet containing 500 mg of micronized purified flavonoid fraction consisting of 450 mg of diosmin and 50 mg of flavonoids expressed as hesperidin.

### Single agents - Active ingredients

### Diosmin/hesperidin (Healthtech Bio Actives S.L.U., Spain, 1308764)

### Ruscus aculeatus extract (Nutraceutica S.r.l., Italy, LIP00338)

### Ananas comosus extract (Nutraceutica S.r.l., Italy, LiP00111)

### Bromelain (Amita Health Care Italia S.r.l., Italy, MPPATT0076)

### Composition

The composition (Vesvein) as reported in Table 1 contains the following active ingredients:
- Micronised bioflavonoids of which 90 % diosmin and 10 % hesperidin (Healthtech Bio Actives S.L.U., Spain, 1308764),
- *Ruscus aculeatus* L. extract (of which 10 % ruscogenin) (Nutraceutica S.r.l., Italy, LIP00338),
- *Ananas comosus* L. extract (tit. 250 GDU/g bromelain) (Nutraceutica S.r.l., Italy, LiP00111), and
- Bromelain 2500 GDU/g (Amita Health Care Italia S.r.l., Italy, MPPATT0076).

**Table 1- Active ingredients of composition of the invention (Vesvein)**

| **Active Ingredients** | **Amount per Tablet (mg)** | **Amount per 2 Tablets (mg)** |
|---|---|---|
| Total bioflavonoids | 500 | 1000 |
| - of which micronized diosmin 450 mg | 450 | 900 |
| - of which micronized hesperidin 50 mg | 50 | 100 |
| *Ruscus aculeatus* extract | 37.5 | 75 |
| - of which ruscogenins | 3.75 | 7.5 |
| -Bromelain (from *Ananas comosus (L.) Merr.) stipes*) 187.5 GDU | 75 | 150 |
| -Pineapple *(Ananas comosus (L.) Merr.) stipes* dry extract) 1.25 GDU | 5 | 10 |
| Total enzymatic activity of Bromelain | 188.75 GDU | 377.5 GDU |

The tablet may further comprise commonly used excipients such as at least one bulking agent (such as microcrystalline cellulose), at least one stabilizer (such as hydroxy-propylmethylcellulose and/or cross-linked sodium carboxy methyl cellulose), at least one anti-caking agent (such as silicon dioxide and/or magnesium salts of fatty acids). Further the tablet may or not be coated. The tablet may comprise a film coating comprising at least one glazing agent (such as hydroxy-propylcellulose and/or polyethylene glycol), at least one coloring agent (such as iron oxide) and optionally at least one sweetener (such as steviol glycosides from Stevia).

Example of Tablet is reported below in Table 2.

**Table 2 - qualitative and quantitative analysis of an exemplified tablet**

| **Ingredients** | **Amount per Tablet (mg)** | **% (w/w)** |
|---|---|---|
| Balanced blend of micronized bioflavonoids comprising | 555.550 | 55.5 |
| -micronized diosmin 450 mg (81%) | | 44.77 |
| -micronized hesperidin 50 mg (9%) | | 5 |
| Microcrystalline cellulose | 185.95 | 18.6 |
| Hydroxypropyl methylcellulose | 87.5 | 8.8 |
| Bromelain (2500 GDU/g) | 75 | 7.5 |
| *Ananas comosus* extract (tit. 250 GDU/g bromelain) | 5 | 0.5 |
| *Ruscus aculeatus* extract (tit. 10% ruscogenine) | 37.5 | 3.8 |
| Cross-linked sodium carboxymethylcellulose | 30 | 3.0 |
| Silicon dioxide | 11.25 | 1.1 |
| Magnesium stearate | 10 | 1.0 |
| Red iron oxide | 2.25 | 0.2 |
| **Unfilmed Tablet core** | **1000** | **100 %** |
| Hydroxypropyl cellulose | 4.37 | 0.43 |
| Polyethylene glycol | 0.51 | 0.05 |
| Steviol glycosides | 0.17 | 0.02 |
| **Total coated tablet** | **1005. 05** | |

Active ingredients were tested alone or in combination (Vesvein or Vesvein double administration) to assess their effectiveness in various parameters including reduction of venous insufficiency after oral treatment. All substances tested were dissolved in 1 ml of Dulbecco's Modified Eagle's Medium (DMEM, Merck Life Science, Rome, Italy) without phenol red and supplemented with 2 mM L-glutamine (Merck Life Science, Rome, Italy), and 1% penicillin-streptomycin (Merck Life Science, Rome, Italy) for all analyses. Substances were diluted 1:2000 to replicate the human dose *in vitro* [23]*.*

Active ingredients quantities for one tablet (i.e. single dose, Vesvein group) (diosmin 450mg, hesperidin 50mg, *Ruscus* extract 37.5mg, *Ananas* extract 5mg, bromelain 75mg) are the following: Diosmin: 0.225 mg/mL, Hesperidin: 0.025 mg/mL, *Ruscus* extract: 0.01875 mg/mL, *Ananas* extract: 0.0025 mg/mL, Bromelain: 0.0375 mg/mL.

Vesvein combination was also evaluated at a double dosage to replicate the effects of double administration (Vesvein Double Administration group). "Double dosage or double administration" refers to the application of a double concentration of the tested formulation (Vesvein) compared to the standard single dose. The cells received twice the amount of active ingredients in a single administration.

This approach was used to simulate the effect of a higher oral intake and to evaluate whether increased doses would have stronger or different biological effects on intestinal and endothelial cells, without inducing toxicity.

Active ingredients quantities for double dose are the following: Diosmin: 0.450 mg/mL, Hesperidin: 0.050 mg/mL, *Ruscus* extract: 0.0375 mg/mL, *Ananas* extract: 0.005 mg/mL and Bromelain: 0.075 mg/mL.

### Cell Culture

Human intestinal epithelial cells, CaCo-2, purchased from the American Type Culture Collection (ATCC, HTB-37^{™}) were cultured in Dulbecco's Modified Eagle's Medium/Nutrient F-12 Ham's (DMEM-F12, Merck Life Science, Rome, Italy) containing 10% foetal bovine serum (FBS), 2 mM L-glutamine and 1% penicillin-streptomycin and kept in an incubator at 37°C and 5% CO2 [24]. For the experiments, cells were used at passage numbers between 26 and 32 to preserve the integrity of paracellular permeability and transport properties [25], maintaining similarity with the intestinal absorption mechanism mimicking what happens after oral intake in humans. According to the experimental protocol, the cells were plated as 1 × 10⁴ cells on 96-well plates to study cell viability using an MTT-based *In vitro* Toxicology Assay Kit (Merck Life Science, Rome, Italy, TOX1-1KT) and radical oxygen species (ROS) production. In addition, 2 × 10⁴ cells were plated on 6.5 mm Transwell^{®} (Corning^{®} Costar^{®}, Merck Life Science, Rome, Italy) with a 0.4 µm pore polycarbonate membrane insert (Corning^{®} Costar^{®}, Merck Life Science, Rome, Italy) in a 24-well plate to perform the absorption analyses [26]. Before treatment, cells were placed in DMEM without red phenol supplemented with 0.5% FBS, 2 mM L-glutamine, and 1% penicillin-streptomycin for 8h to synchronise them and then incubated at 37°C with 5% CO2.

Human umbilical vein endothelial cells (HUVEC) purchased from ATCC (Manassas, VA, USA, ATCC number CRL-1730, Cat. No. PCS-100-010 ) were cultured in 0.1% gelatincoated flask with Endothelial Growth Medium-2 (EGM-2) containing 2% FBS, 0.04% hydrocortisone, 0.4% hFGF-B, 0.1% VEGF, 0.1% R3-IGF-1, 0.1% ascorbic acid, 0.1% hEGF, 0.1% GA-1000, 0.1% heparin (all from Lonza, Walkersville, MD, USA) and maintained at 37 °C and 5% CO2 as previously described [27]. HUVEC cells were incubated at 37° C in a 95% humidified atmosphere with 5% CO2 until passage 3 to 6 before being used in the experiments [27]. Therefore, 3 x 10⁴ cells were placed in 96-well plate in a complete growth medium to perform cell viability analysis by MTT test and ROS production analysis. In addition, 7 x 10⁴ cells/well were plated for 48 hours in 24-well plates previously coated with Matrigel (BD Biosciences; incubated for 30 minutes at 37°C) to analyse the mechanisms involved in venous insufficiency [28]. Specifically, cells placed on Matrigel were treated with KCl to emulate the characteristics of vessel physiology during venous insufficiency due to increased extracellular matrix (ECM) destruction and venous pressure [10].

### Experimental protocol

The study was partitioned into two distinct phases: an intestinal phase and a venous phase. As an initial endeavour within the intestinal phase, the assessment of cell viability (utilising the MTT assay) and the quantification of ROS production (employing Cytochrome C reduction) in intestinal cells subjected to treatment with diosmin/hesperidin, *Ruscus aculeatus* L. extract, *Ananas comosus* extract, bromelain, and their combination over a period ranging from 1 to 6 hours was conducted; furthermore, the aforementioned combination was also administered at a dosage that was twice the standard amount. Subsequently, the examination of the absorption and transport mechanisms of both single- and double-dose compositions, as well as individual agents, was performed through *in vitro* reconstruction of the intestinal barrier utilising a three-dimensional model, which has been validated in existing literature [29] and approved by the European Medicines Agency (EMA) and the Food and Drug Administration (FDA) [30,31]. Permeability analysis (assessment of the apparent permeability coefficient, Papp), measurements of transepithelial electrical resistance (TEER) values, uptake analysis and tight junction (TJ) activity were conducted on the 3D model. In the venous phase, metabolised intestinal cells from the 3D model that crossed the intestinal barrier could directly stimulate HUVEC venous cells placed in the basolateral compartment on a Matrigel layer to recreate an *in vitro* vein model. The *in vitro* vein model was treated with all test substances for 24 h and then damaged for 15 min with 96 mM KCl [10]. The degree of cell viability, endothelial cell metabolic activity, ATP production, and oxidative/inflammatory status, which are essential for proper vein function, were analysed. In addition, all mechanisms involved in the processes of maintaining vessel tone were evaluated by assessing elastin following vessel contraction/relaxation movements, vessel wall permeability by analysing tissue integrity (measurement of TEER), and the leading promoters of vessel permeability such as Endothelin-1 (ET-1) and eNOS resulting in the production of NO as a vasodilating agent. Finally, the ability of the agents under investigation to maintain vessel architecture was analysed by evaluating the significant cadherins involved, such as VEGF and MMP-9, by Western Blot analysis.

The study samples were tested in comparison with a known Commercial product.

### Cell viability

The MTT (A3-(4,5-Dimethylthiazol-2-yl)-2,5-Diphenyltetrazolium bromide) test was performed following a standard protocol [32] to exclude cytotoxic effects. Briefly, after stimulations, both CaCo-2 and HUVEC cell types were incubated with 1% MTT dye (Merck Life Science, Milan, Italy, Cat. No: M5655) in white DMEM for 2 h at 37°C in an incubator. Purple formazan crystals were dissolved in an equal volume of solubilising solution. Cell viability was determined by measuring the absorbance at 570 nm with correction at 650 nm, using a spectrometer (Infinite 200 Pro MPlex, Tecan, Milan, Italy), and calculated by comparing the results with control cells without any stimulus (baseline 0%).

### Reactive Oxygen Species production

Quantification of superoxide anion release was obtained following a standard protocol based on cytochrome C reduction [33], and the absorbance in the culture supernatants was measured at 550 nm using a spectrophotometer (Infinite 200 Pro MPlex, Tecan, Männedorf, Switzerland). Specifically, 100 µL of cytochrome C (Merck Life Science, Milan, Italy) was added to all wells, while 100 µL of superoxide dismutase (Merck Life Science, Milan, Italy) and 100 µL of cytochrome C were added to the empty wells; the plate was then incubated for 30 min. The O2 rate was expressed as the mean ± SD (%) of nanomoles of reduced cytochrome C per microgram of protein compared to the control (line 0).

### Intestinal barrier in vitro model

An *in vitro* intestinal barrier model was created using the Transwell^{®} system, following a standard protocol reported in the literature [34] and approved by EMA and FDA to predict the absorption, metabolism and bioavailability of different substances after oral intake in humans [30,31]. Briefly, CaCo-2 cells, plated as described above, were maintained in a complete culture medium, changing it every other day on the basal and apical sides for 21 days before the simulations [34]. Throughout the maturation period, TEER values were evaluated by EVOM3, coupled with STX2 rod electrodes (World Precision Instruments, Sarasota, FL, USA), to assess the formation of mature intestinal epithelia and a proper paracellular mechanism. Absorption analysis began on day 21 when TEER values were ≥ 400 Ω·cm2 [35]. Before stimulation, on the apical side the culture medium was adjusted to pH 6.5, corresponding to the pH of the lumen of the small intestine, while on the basolateral side the culture medium was adjusted to pH 7.4, corresponding to the pH of the blood [25]. Under these experimental conditions, substances were systematically introduced to the apical environment within a temporal framework (ranging from 2 to 6 hours). At each specified time interval, intestinal absorption or bioavailability was evaluated by applying 0.04% fluorescein (Merck Life Science, Milan, Italy), a fluorescent marker dye utilised to quantify transepithelial transport [36]. The amount of fluorescein transported was measured at 37°C for 40 minutes by incubating Caco-2 cells at the above concentration (apical pH, 6.0; basolateral pH, 7.4). Fluorescence was detected with a fluorescence spectrophotometer (Infinite 200 Pro MPlex, Tecan, Männedorf, Switzerland) at excitation/emission wavelengths of 490/514 nm. The cells were stimulated with all substances from 2 to 6 hours before subsequent analyses, including the permeability assay. The data obtained were converted into absorption using the formula for the permeability index: 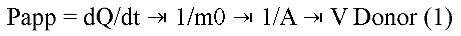
dQ: amount of substance transported (nmol or µg);
dt: incubation time (s);
m0: amount of substrate applied to the donor compartment (nmol or µg);
A: Transwell^{®} membrane surface area (cm2);
VDonor: volume of the donor compartment (cm3).

Negative cell-free controls were analysed to exclude the influence of the Transwell^{®} membrane. The analysis was performed in triplicate and reproduced five times.

### TJs Analysis

The lysates derived from CaCo-2 cells were utilised to assess the concentration of Occludin utilising the Human Occludin (OCLN) ELISA Kit (MyBiosource, San Diego, CA, USA, MBS2704294), the level of claudin-1 via the ELISA Kit (Cusabio Technology LCC, Houston, TX, USA), and the measurement of Zona Occludens 1 (ZO-1) through the human tight junction protein 1 (TJP1) ELISA kit (MyBiosource, San Diego, CA, USA), following the guidelines provided by the manufacturer [37]. The absorbance was quantified utilising a Tecan spectrophotometer at a wavelength of 450 nm. Data were derived by juxtaposing them against the standard curve, which ranged from 0 to 1500 pg/mL for Occludin and from 0 to 1000 pg/mL for both claudin-1 and ZO-1. The findings were articulated as a percentage (%) relative to the control (0 line), which consisted of five distinct experiments executed in triplicate.

### Gut-veins axis

To establish the intestinal-vein axis, Caco-2 intestinal cells were cultivated on the semipermeable membrane of a Transwell^{®} apparatus for 21 days [34]. On the 19th day of the intestinal barrier maturation process, HUVEC cells were introduced into their respective culture medium at the basolateral compartment of the Transwell^{®} and subj ected to incubation for 48 hours at a temperature of 37°C, with an atmosphere consisting of 5% CO2 and 95% humidity. To facilitate the creation of the gut-vein axis through co-culture, the Transwell^{®} insert containing the matured intestinal monolayer was subsequently placed into a 24-well plate that housed the HUVECs on a Matrigel substrate. A proper maturation time of 7 × 10⁴ HUVEC cells in the vein 3D model was determined by TEER with EVOM3 coupled with STX2 chopstick electrodes (World Precision Instruments, Sarasota, FL, USA), until the cells reached a threshold of 20 Ω ▪ cm2.

### Determination of ATP production

After each stimulation, the medium was extricated, and the cells were promptly treated with the constituents of the ATP assay kit (Producer: Merck Life Science/Sigma-Aldrich Ct. No. SCT149, nucleotide releasing buffer, ATP monitoring enzyme, enzyme reconstitution buffer, ATP), adhering strictly to the manufacturer's specifications. Luminescence was quantified one minute after the introduction of the ATP monitoring enzyme utilizing a multilabel plate reader (Infinite 200 Pro MPlex, Tecan, Männedorf, Switzerland), and the luminescence data was articulated as means ± SD% of µmol of ATP/g protein [38].

### Tumor Necrosis Factor α (TNFα) Assay Kit

According to the manufacturer's instructions, TNFα concentration in HUVEC cells supernatant was determined using the TNFα ELISA kit (Merck Life Sciences, Milan, Italy EZHTNFA-150K). The plate, in which 50 µL of HUVEC cell supernatant was dosed, was incubated at 37°C for 60 minutes, following the introduction of 100 µL of biotin-conjugated anti-bodies into each well. At the end of the incubation period, the wells were thoroughly rinsed three times with a designated wash buffer, followed by the addition of 100 µL of SABC working solution. After a 30-minute incubation at 37°C, each well underwent a cleaning process and 90 µL of TMB substrate was introduced. After 10-20 minutes, 50 µL of stop solution was added to each well. The optical density at 450 nm was measured using a plate reader (Infinite 200 Pro MPlex, Tecan, Männedorf, Switzerland); the colour intensity (optical density; OD) and the concentration of the standard (ranging from 0 to 6000 pg/mL) were correlated by applying standard curves [39]. The results of five independent tests conducted in triplicate were presented as mean ± SD (%) versus control (0 lines).

### Interleukin 1β (IL-1β) Assay Kit

IL-1β present in HUVEC lysates was measured following the guidelines provided by the manufacturer, utilising an IL-1β ELISA kit (R&D Systems, Minneapolis, MN, USA, DLB50). The methodology entailed the introduction of IL-1β-supplemented culture medium into a microplate strip (100 µL/well), followed by incubation for 2 hours at room temperature, subsequent mixing with IL-1β conjugate, and additional incubation for an equivalent period at room temperature. Comprehensive washes were conducted both before and following the two incubation phases. Thereafter, 100 µL of substrate solution was incorporated to facilitate the development of chemiluminescence, and the absorbance was assessed using a microplate reader (Infinite 200 Pro MPlex, Tecan, Männedorf, Switzerland) at a wavelength of 450 nm, with a correction implemented at 570 nm. The quantification of IL-1β involved comparing the sample readings against the standard curve that was generated [40].

### Human Elastin ELISA Kit

The Human Elastin ELISA kit (MyBiosource, San Diego, CA, USA, MBS033425) was employed to quantify elastin levels in HUVEC cell lysate following the protocols delineated by the manufacturer. In summary, 100 µL of the sample was dispensed into a well pre-coated with a specific antibody, followed by an incubation period of 2 hours at a temperature of 37°C. Upon the incubation period conclusion, the wells contents were discarded, and 100 µL of Biotin antibody was introduced for an additional incubation of 1 hour at 37°C. Following this incubation, the well contents were removed, and the wells underwent a thorough washing procedure three times with a 1x Wash Solution before adding 100 µL of HRP-avidin. After a subsequent incubation of 1 hour at 37°C, the plate was subjected to five wash cycles, and then 90 µL of TMB Substrate was incorporated into the wells for a 15-30 min incubation at 37°C. After completing this step, 50 µL of Stop Solution was added to each well, and the absorbance of the plate was measured at 450 nm using a spectrophotometer (Infinite 200 Pro MPlex, Tecan, Männedorf, Switzerland). The results were derived by juxtaposing the obtained data with the standard curve (0 - 200 pg/mL). They were articulated as a percentage (%) relative to the control (line 0) derived from five independent experiments conducted in triplicate.

### Human Endothelin-1 ELISA Kit

Human Endothelin-1 (ET-1) ELISA Kit (ThermoFisher, Waltham, Massachusetts, USA) was used according to the manufacturer's instructions to quantify the amount of Endothelin-1 in the supernatant of HUVEC cells. Briefly, 50 µL of samples were dispensed into wells, and the plate was incubated at room temperature for 1h. At the end of incubation, the material in the wells was removed, wells were washed with 1x wash solution 4 times and 50 µL of Endothelin-1 Conjugate was put into the wells. After an incubation of 1h, wells were washed 4 times and 100 µL of TMB Substrate Solution was added to each well. After 30 minutes of incubation at room temperature, 50 µL of Stop Solution was placed in the well, and the plate was read at 450 nm with a spectrophotometer (Infinite 200 Pro MPlex, Tecan, Männedorf, Switzerland). The results were obtained by comparing the data with the standard curve (ranging from 0 to 100 pg/mL). They were expressed as the percentage (%) against the control (line 0) of five independent experiments performed in triplicate.

### Human eNOS/NOS3 ELISA Kit

eNOS concentration was measured following the manufacturer's instructions of DuoSet ELISA kit for Human eNOS (R&D Systems, Minneapolis, MN, USA, DY950-05) in HUVEC cells. Using a spectrometer (Infinite 200 Pro-MPlex, Tecan, Männedorf, Switzerland), the absorbance of the samples was measured at 450 nm. The results were displayed as a percentage (%) normalized to the untreated samples in reference to the standard curve (78.1 - 5,000 pg/mL) created using a standard. The results were shown as mean ± SD (%) vs. control (line 0) after five different tests were run in duplicate.

### Nitric Oxide production

Following the stimulation procedures, Griess' reagent (Promega, Italy, G2930) was introduced to the supernatants derived from HUVEC cells to evaluate the production of nitric oxide (NO) [41]. After stimulation, 50 µL of supernatant was extracted from each well and transferred into a new 96-well plate containing 50 µL of sulfanilamide solution. The plate was subjected to incubation at a temperature of 37°C in a dark environment for 10 minutes. In the concluding step, 50 µL of N-1-naphthyl ethylenediamine dihydro-chloride (NED solution) was added to each well, followed by an additional incubation period of 10 minutes at 37°C in darkness. Upon completion of the incubation, a spectrophotometric measurement (Infinite 200 Pro MPlex, Tecan, Männedorf, Switzerland) was conducted at a wavelength range of 520-550 nm within a time frame of 30 minutes. This assay can detect NO2-, recognised as one of nitric oxide's most enduring and least volatile derivatives. The results are expressed as a percentage (%) normalised to the untreated control samples, utilising a standard nitrate calibration curve ranging from 0 to 100 µM.

### Western Blot Analysis

At the end of each stimulation, keratinocytes were rinsed with cold 1× PBS (Merck Life Science, Rome, Italy) and lysed using Complete Tablet Buffer (Roche, Basel, Switzerland, 04693132001) enriched with 2 mM sodium orthovanadate (Na3VO4), 1 mM phenylme-thanesulfonylfluoride (PMSF) (Merck Life Science, Rome, Italy 52332), a 1:50 phosphatase inhibitor mix (250 mM Sodium Fluoride, 50 mM β-Glycerophosphate (Cat. No. 35675), 50 mM Sodium Pyrophosphate Decahydrate, 50 mM Sodium Orthovanadate) (Merck Life Science, Rome, Italy, 524629), and a 1:200 protease inhibitor mix (Merck Life Science, Rome, Italy, Cat. No: P8340, Mix components: AEBSF (4-(2-Aminoethyl) benzenesulfonyl fluoride) - serine protease, Aprotinin - serine protease, Bestatin - aminopeptidase, E-64 - cysteine protease, Leupeptin - serine and cysteine protease, Pepstatin A - aspartate protease) to yield a total protein extract that underwent laboratory analysis: a 1:50 phosphatase inhibitor mix (Merck Life Science, Rome, Italy) and a 1:200 protease inhibitor mix (Merck Life Science, Rome, Italy) to produce a total protein extract that was centrifuged at 14,000× g for 20 minutes at 4 °C. Subsequently, 40 µg of protein per extract was applied to 8% and 10% SDS-PAGE gels and transferred onto a polyvinylidene difluoride (PVDF) membrane, which was incubated overnight with specific primary antibodies such as MMP9 (1:500, Santa Cruz, CA, USA, Cat. No: sc-21733) and VEGF (1:500, Santa Cruz, CA, USA, Cat. No: sc-7269). The expression levels of all proteins were normalised and confirmed by detecting β-actin (1:5000, Merck Life Science, Rome, Italy, Cat. No: A5441) and presented as mean ± SD (%) relative to the control value (line 0).

### Statistical analysis

One-way analysis of variance (ANOVA) and Bonferroni post hoc tests were used to process the data acquired using Prism GraphPad statistical software 9.4.1. A Student's t-test with two tails was adopted to compare the two groups. A two-way ANOVA was conducted to evaluate multiple group comparisons, followed by a two-sided Dunnett *post hoc* test. The mean ± SD of at least five independents performed in triplicate variables was used to express all results.

### Example 1: Safety evaluation of Vesvein and individual agents on intestinal epithelium to exclude toxicity

A cell viability assay and an assessment of ROS production at the intestinal level were conducted to evaluate the safety profile of the various ingredients and composition for a treatment period ranging between 1 and 6 hours to rule out any potential cytotoxic effects. More specifically the effect of single ingredients as well as the combination of diosmin/hesperidin, *Ruscus* extract, bromelain, and *Ananas* extract (designated as Vesvein) were examined at the intestinal level and compared against a commercially available product containing only the combination of diosmin/hesperidin. Furthermore, Vesvein combination was also evaluated at a double dosage to replicate the effects of double administration (Vesvein Double Administration). As shown in Figure 1A, all individual agents increased cell viability, with a peak effect at 4h of treatment compared with the control (untreated cells, 0%-line, p<0.05) similar to the commercial product. The composition of the present invention (Vesvein) was able to significantly increase cell viability compared with the control group and with single agents (p<0.0001). Furthermore, the composition of the present invention outperformed the commercial product at 4h of treatment by about 63% (p<0.0001). These results support a synergistic effect of the single ingredients. In addition, the double dosage of Vesvein showed greater beneficial effects than the control group and single agents throughout the treatment period. The maximum effect of Vesvein Double Administration was achieved at 4h of treatment and outperformed Vesvein by about 6% and the commercial competitor by about 65% (p<0.0001). To summarise, viability data showed that Vesvein and Vesvein Double Administration were safe for intestinal cells. Further Vesvein Double Administration improved cell viability compared to control group, individual ingredients, commercial product, and Vesvein (p<0.0001). Similar results were observed for ROS production (Figure 1B). Specifically, all individual agents maintain ROS levels below the control value (1h to 4h of treatment) or below the threshold values (5h to 6h). The term "Below the control value (1h to 4h)" means that, in the first 4 hours of treatment, the single active agents were able to reduce the production of ROS (reactive oxygen species) below the level observed in untreated cells (= control). This indicates an active and early antioxidant effect. The term "Below the threshold values (5h to 6h)" refers to a threshold value identified as a maximum acceptable limit of oxidative stress that does not compromise cell viability or the functionality of the intestinal barrier. Even if it is no longer reduced below the control level, the ROS levels remain within safe limits. Thus, in the first 4 hours the single agents lower the ROS level below the control reference value, and between the fifth and sixth hour the ROS levels slightly increase, remaining below a threshold considered tolerable/non-toxic, meaning that the antioxidant effect is still present, although less marked.

Individual agents exerted similar effects to the commercial product. In contrast, the most significant beneficial effect was observed after single or double administration of Vesvein compared with the individual agents and the commercial product (p<0.05), with no significant difference between the two different administrations.

### Example 2: Integrity and permeability analysis on a 3D model of intestinal barrier in vitro

To evaluate the permeability and transport of Vesvein, Vesvein Double Administration and individual components, further experiments were performed on an *in vitro* 3D model of the intestinal barrier that mimics as much as possible the *in vivo* setting. In this context, all substances were tested from 1h to 6h to measure TEER values, Papp and absorption rate.

In the present Example, the "absorption rate" coincides with the percentage values of absorption reported in Figure 2F, calculated from the Papp values of Figure 2E with the formula:

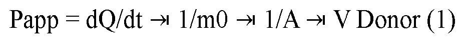

These percentage values represent how much substance managed to cross the intestinal barrier based on time, therefore they reflect the speed and quantity of absorption.

In addition, tight junction protein lysate levels were assessed to confirm the integrity of the intestinal monolayer following treatment with all tested agents. As illustrated in Figure 2A, TEER values obtained during the treatment duration show that individual agents and their combinations are capable of maintaining TEER values above control values. Furthermore, Vesvein and Vesvein Double Administration demonstrate a superior efficacy in preserving TEER values compared to individual agents and to Commercial product (p<0.0001), supporting an enhancement of the ionic flux of paracellular exchanges across the intestinal epithelium. These findings were corroborated by the assessment of TJ protein levels, including Claudin-1, Occludin, and ZO-1. Indeed, as depicted in Figures 2B-D, all tested individual agents increase lysate levels of Claudin-1, Occludin, and ZO-1 in comparison to the control and similar to the Commercial product. Vesvein and Vesvein Double Administration enhance Claudin-1 lysate levels (Fig. 2B) by approximately 39% and 45% compared to diosmin/hesperidin (p<0.0001), 50% and 55% compared to *Ruscus aculeatus* (p<0.0001), 32% and 38% compared to *Ananas comosus* (p<0.0001), 37% and 42% compared to bromelain (p<0.0001), and about 41% and 47% compared to the Commercial product (p<0.0001), respectively. Additionally, Occludin lysate levels (Fig. 2C) are also significantly augmented following treatment with Vesvein and Vesvein Double Administration by approximately 56.5% and 62% compared to diosmin/hesperidin (p<0.0001), 64% and 69% compared to *Ruscus aculeatus* (p<0.0001), 53% and 59% compared to *Ananas comosus* (p<0.0001), 50% and 57% compared to bromelain (p<0.0001), and about 59% and 64% compared to the Commercial product (p<0.0001). Lastly, Vesvein and Vesvein Double Administration increase ZO-1 lysate levels (Fig. 2D) by approximately 26.5% and 41% vs diosmin/hesperidin (p<0.0001), 36% and 48% vs *Ruscus aculeatus* (p<0.0001), 25% and 39% vs *Ananas comosus* (p<0.0001), 27.5% and 41.5% vs bromelain (p<0.0001), and by approximately 21% and 36% vs Commercial product (p<0.0001). Moreover, permeability and absorption analysis (Figure 2E-F) encompassing all tested agents was performed. Vesvein and Vesvein Double Administration exhibited superior uptake kinetics compared to the individual agents and the Commercial product (p<0.0001).

### Example 3: Effect of individual components and combinations on 3D vein model

Building on the data collected at the intestinal level, subsequent experimental frameworks were conducted utilising a three-dimensional vascular model established in direct correlation with intestinal cells. The model aimed at reconstructing the gut/vascular axis and at elucidating the direct cellular interactions involved, thereby aiding in the evaluation of the effects of all tested molecules. To replicate *in vitro* the distinct characteristics associated with chronic venous disease insufficiency, the HUVEC employed in fabricating the three-dimensional vascular model was subjected to a 15-minute exposure to 96 mM KCl following treatment with all tested ingredients. KCl is an inducer that activates specific mechanisms contributing to elevated ECM degradation and increased venous pressure [10]. As shown in Figure 3, all tested agents almost restore cell viability to control values and reduce oxidative stress by counteracting the adverse effects of KCl. Specifically, in Figure 3A, the effects of individual agents and combinations on KCl-induced CVD condition are shown. After damage with KCl, venous cell viability was drastically reduced (about 9%) compared with control (untreated cells, p<0.05). Cells treated with the individual agents resist KCl-induced damage with an increase in cell viability of 67% (diosmin/hesperidin vs KCl, p<0.05), 83% (*Ruscus aculeatus* vs KCl, p<0.05), 92% (*Ananas comosus* vs KCl, p<0.05), 83% (Bromelain vs KCl, p<0.05), 2.76-fold (Commercial product vs KCl, p<0.05), 3.43-fold (Vesvein vs KCl, p<0.05) and 3.68-fold (Vesvein Double Administration vs KCl, p<0.05). The most significant protective effect was observed with treatment with Vesvein and Vesvein Double Administration compared with the single agents. Moreover, Vesvein and Vesvein Double Administration performed better than the Commercial product as they were able to increase cell viability by about 27% (p<0.05) and 34% (p<0.05). Similarly, the beneficial protective effect of Vesvein and Vesvein Double Administration and the single agents was also observed on ATP production (Figure 3B). Specifically, KCl significantly reduced ATP production compared with control (p<0.05). However, Vesvein and Vesvein Double Administration treatment restored ATP production compared with KCl-induced damage by approximately 1.56-fold and 1.72-fold (p<0.05). In addition, ROS production (Fig. 3C) was evaluated to test the antioxidant effect of the individual tested agents and combinations against KCl-induced damage. More specifically, following damage induction with KCl, ROS production was significantly increased (about 14.5%) compared with control (p<0.05). Surprisingly, cells treated with the individual agents resist KCl-induced damage by reducing ROS production by 41% (diosmin/hesperidin vs KCl, p<0.05), by 45% (*Ruscus aculeatus* vs KCl, p<0.05), by *50% (Ananas comosus* vs KCl, p<0.05), by 46% (Bromelain vs KCl, p<0.05), by 83% (Commercial product vs KCl, p<0.05), by 90% (Vesvein vs KCl, p<0.05) and by 95% (Vesvein Double Administration vs KCl, p<0.05). However, compared with single agents, the most significant protective effect was observed with treatment with Vesvein and Vesvein Double Administration. Finally, to confirm the beneficial effect of the compositions of the invention, the inflammatory response induced by KCl was analysed by testing the production of inflammatory cytokines: tumour necrosis factor-α (TNFα) (Figure 3D) and IL-1β (Figure 3E). Specifically, individual agents and the Commercial product reduce the release of inflammatory cytokines below the levels produced by damage with KCl (p<0.05). The most significant beneficial effect on the reduction of TNFα and IL-1β production was observed after treatment with Vesvein and Vesvein Double Administration, reducing TNFα production by approximately 89% and 95% compared with KCl (p<0.05). Vesvein and Vesvein Double Administration decreased IL-1β production by approximately 90.5% and 94% compared with KCl group (p<0.05). Then Vesvein and Vesvein Double Administration combinations maintain venous cells well-being in a condition of venous insufficiency induced by KCl treatment.

### Example 4: Effect of individual components and combinations on maintaining venous integrity and tone

Further analyses were conducted concerning the maintenance of vascular integrity, regulation of vascular contraction and relaxation dynamics, neovascularisation, and evaluations of vascular permeability and vasodilation. As an initial examination, TEER was quantified in cells subjected to all tested agents while placed in a compromised state induced by KCl (Figure 4A). Data analysis revealed that, notwithstanding the capacity of the individual agents to increase TEER measurements above those shown for KCl-induced damage (p<0.05), they could not restore TEER values to exceed the control measurements. Conversely, Vesvein and Vesvein Double Administration combinations, as well as the Commercial product, elevate TEER values above those induced by KCl (p<0.05) and also above control values (p<0.05). Further, to determine whether the tested agents might be able to exert a regulatory effect on vessel contraction and relaxation movements, Elastin lysate levels were assessed after treatment with all tested molecules and after induction of damage with KCl (Figure 4B). Data showed that KCl reduced Elastin levels by approximately 11.4% compared with control (p<0.05). At the same time, all tested agents reverse this effect and restore elastin levels to control levels. Specifically, individual agents restore Elastin levels relative to KCl by about 60.5% (Diosmin/hesperidin, p<0.05), by about 78% (*Ruscus aculeatus,* p<0. 05), about 82% (*Ananas comosus,* p<0.05), about 78% (Bromelain, p<0.05), about 1.26-fold (Vesvein, p<0.05), about 1.52-fold (Vesvein Double Administration, p<0.05) and about 1.20-fold (Commercial product, p<0.05). Furthermore, Vesvein Double Administration exert a significant beneficial effect when compared to the Commercial product by about 61% (p<0.05). Finally, Endothelin-1 (Fig. 4C) was examined as a potential biomarker for neovascularisation by induction an angiogenic phenotype in the cellular model. As illustrated in Figure 4C, KCl-induced cytotoxicity resulted in a reduction of Endothelin-1 cell media concentrations by approximately 7.5% when compared to the control group (p<0.05); conversely, the administration of diosmin/hesperidin, *Ruscus aculeatus, Ananas comosus,* bromelain, Vesvein, Vesvein Double Administration and the Commercial product elevate Endothelin-1 levels relative to KCl-induced damage by approximately 53%, 56.6%, 57%, 50%, 2.2-fold, 2.7-fold, and 2.04-fold, respectively (p<0.05). Vesvein Double Administration displayed a significant advantageous outcome in respect to the Commercial product, enhancing endothelin-1 levels by approximately 40% relative to the Commercial product (p<0.05). Collectively, these findings highlight the efficacy of Vesvein and Vesvein Double Administration combination in regulating the mechanisms governing vascular contraction, relaxation, and neovascularisation.

Recently, it has been proposed that CVD also has a molecular cause due to increased expression of MMPs that promote venous wall dilatation [42]. This increase may be preceded by an inflammatory process contributing to venous degeneration and endothelial dysfunction [10]. VEGF maintains vascular integrity and responsiveness in this context by mediating NO, vasopermeability and dilatory responses. VEGF is one of the most critical factors in angiogenesis and influences the activity of endothelial and inflammatory cells [43]. VEGF stimulates eNOS expression in endothelial cells and thus steadily increases NO formation [16]. As reported Figure 5A, KCl-induced damage stimulates MMP-9 release by about 11.65% compared with control (p<0. 05). Individual tested agents prevent ECM degradation and thus venous degeneration by reducing MMP-9 levels by about 63.5% (Diosmin/hesperidin, p<0.05), 59% (*Ruscus aculeatus,* p<0. 05), 60.5% (*Ananas comosus,* p<0.05), and 50.6% (Bromelain, p<0.05) compared with KCl-induced damage. Vesvein and Vesvein Double Administration reduce MMP-9 levels to a greater extent than single agents (2.35-fold and 2.76-fold vs diosmin/hesperidin, p<0.05; 2.21-fold and 2.55-fold vs *Ruscus aculeatus,* p<0.05; 2.25-fold and 2.58-fold vs *Ananas comosus,* p<0.05; 2-fold and 2.26-fold vs bromelain, p<0.05), similarly or exceeding the effect of the Commercial product. As mentioned earlier, VEGF also appears to be an important marker for vascular reactivity, so this was also evaluated following treatment with the tested agents. In Figure 5B, VEGF levels are drastically reduced compared with control (-10.45%, p<0.05) after KCl treatment. VEGF levels were found to be restored after treatment with the individual agents. However, the most significant recovery was observed after treatment with Vesvein Double Administration, which showed a substantial effect of approximately 64% compared with the Commercial product (p<0.05). Next, eNOS levels (Figure 5D) and NO production (Figure 5C) were assessed following treatment and damage induction. Data obtained showed that Vesvein Double Administration increased NO production to a greater extent than the individual agents (1.86-fold vs diosmin/hesperidin, p<0.05; 1.74-fold vs *Ruscus aculeatus,* p<0.05; 1.66-fold vs *Ananas comosus,* p<0.05; 1.68-fold vs bromelain, p<0.05) and the Commercial product (54%, p<0.05). This increase was found to be a consequence of increased eNOS levels at the cellular level after treatment with the tested agents. Vesvein Double Administration increased eNOS levels to a greater extent than the individual agents (1.34-fold vs diosmin/hesperidin, p<0.05; 1.26-fold vs *Ruscus aculeatus,* p<0.05; 1.21-fold vs *Ananas comosus,* p<0.05; 1.22-fold vs bromelain, p<0.05), the Commercial product (65%, p<0.05).

In Figure 5E, a western blot analysis shows that MMP-9 and VEGF bands are more intense after Vesvein or Vesvein Double Administration compared to the single ingredients and the commercial product. Vesvein and Vesvein Double Administration enhance the expression of these two proteins.

### Example 5: Composition of the invention and its effect on the pathogenic mechanism of venous disease

Overall, the present data demonstrate that the composition of the present invention (Vesvein) has a similar activity or perform better than another commercial product (full daily dose) on all evaluated parameters. The composition of the invention provides significant benefits, as shown in Table 3.

**Table 3- Overall view of Vesvein and Vesvein double administration results**

| | 1 dose vs other commercial product | 2 doses vs other commercial product | 2 doses vs 1 dose |
|---|---|---|---|
| **FEELING OF LEG HEAVINESS, TIRED LEGS** | | | |
| NO production | Equal | Better | Better |
| eNOS | Equal | Better | Better |

| **SWOLLEN LEGS, ANKLES AND FEET** | | | |
|---|---|---|---|
| TEER | Equal | Equal | Equal |
| Elastin | Equal | Better | Better |
| Endothelin | Equal | Better | Better |
| Ratio MMP9/beta actin | Equal | Equal | Equal |
| VEGF/B actin | Equal | Better | Better |

| **PAINFUL LEGS** | | | |
|---|---|---|---|
| ROS | Better | Better | Equal |
| TNFa | Better | Better | Equal |
| IL1b % | Better | Better | Equal |
| ATP production % | Equal | Equal | Equal |
| Cell viability % | Better | Better | Equal |

### Example 6: Clinical Effects of the compositions of the invention

### Material and methods

This study evaluates the effects of the combination of diosmin, hesperidin, bromelain, *Ananas* extract and *Ruscus* extract on the quality of life of subjects having symptoms compatible with early stages of venous disease, in particular CVD. The subjects may have lower extremities disorders possibly prodromal of CVD. The combination is administered for 12 weeks and the quality of life of subjects with symptoms possibly attributable to early stages of chronic vascular disease is monitored as outlined in Fig. 6 and study design below.

### Composition

Subjects enrolled in the study take one tablet as described in Table 4 twice a day for 12 weeks.

**Table 4 - Active ingredients of the tablet (Vesvein)**

| **Active Ingredients** | **Amount per Tablet (mg)** | **Amount per 2 tablets (mg)** |
|---|---|---|
| Total bioflavonoids | 500 | 1000 |
| - of which micronized diosmin 450 mg | 450 | 900 |
| - of which micronized hesperidin 50 mg | 50 | 100 |
| *Ruscus aculeatus* extract | 37.5 | 75 |
| - of which ruscogenins | 3.75 | 7.5 |
| -Bromelain (from *Ananas comosus (L.) Merr.) stipes*) 187. 5 GDU | 75 5 | 150 10 |
| -Pineapple *(Ananas comosus (L.) Merr.) stipes* dry extract) 1.25 GDU | | |
| Total enzymatic activity of Bromelain | 188.75 GDU | 377.5 GDU |

The tablet may further comprise commonly used excipients such as at least one bulking agent (such as microcrystalline cellulose), at least one stabilizer (such as hydroxy-propylmethylcellulose and/or cross-linked sodium carboxy methyl cellulose), at least one anti-caking agent (such as silicon dioxide and/or magnesium salts of fatty acids). Further the tablet may or not be coated. The tablet may comprise a film coating comprising at least one glazing agent (such as hydroxy-propylcellulose and/or polyethylene glycol), at least one coloring agent (such as iron oxide) and optionally at least one sweetener (such as steviol glycosides from Stevia).

Example of Tablet is reported below in Table 5.

**Table 5 - qualitative and quantitative analysis of an exemplified tablet**

| **Ingredients** | **Amount per Tablet (mg)** | **% (w/w)** |
|---|---|---|
| Balanced blend of micronized bioflavonoids comprising | 555.550 | 55.5 |
| -micronized diosmin 450 mg (81%) | | 44.77 |
| -micronized hesperidin 50 mg (9%) | | 5 |
| Microcrystalline cellulose | 185.95 | 18.6 |
| Hydroxypropyl methylcellulose | 87.5 | 8.8 |
| Bromelain (2500 GDU/g) | 75 | 7.5 |
| *Ananas comosus* extract (tit. 250 GDU/g bromelain) | 5 | 0.5 |
| *Ruscus aculeatus* extract (tit. 10% ruscogenine) | 37.5 | 3.8 |
| Cross-linked sodium carboxymethylcellulose | 30 | 3.0 |
| Silicon dioxide | 11.25 | 1.1 |
| Magnesium stearate | 10 | 1.0 |
| Red iron oxide | 2.25 | 0.2 |
| **Unfilmed Tablet core** | **1000** | **100 %** |
| Hydroxypropyl cellulose | 4.37 | 0.43 |
| Polyethylene glycol | 0.51 | 0.05 |
| Steviol glycosides | 0.17 | 0.02 |
| **Total coated tablet** | **1005. 05** | |

### Endpoints

### Primary Endpoint

The primary endpoint is the difference between the total score obtained on the CIVIQ-20 questionnaire collected at visit 3 (12 weeks from the administration of the combination, V3 visit) compared to baseline. The endpoint is achieved if there is a reduction in the questionnaire score of at least 5 points from the baseline score after 12 weeks of treatment. The CIVIQ-20 questionnaire or "Chronic Venous Insufficiency quality of life Questionnaire" refers to a 20-item questionnaire administered to chronic venous disease (CVD) patients to assess quality-of-life providing a global index and an outline of 4 quality-of-life dimensions: "pain" (4 items), "physical" (4 items), "psychological" (9 items), and "social" (3 items) in patients with chronic venous disease. Items on the CIVIQ-20 scale are scored from 1 to 5. A low score corresponds to greater patient comfort.

### Secondary Endpoints

The secondary endpoints are:
- any changes in the total score obtained on the CIVIQ-20 questionnaire recorded at the visit 2 (4 weeks of taking the composition of the invention, V2 visit) compared to the score obtained at baseline. The endpoint is considered reached in the event of a reduction in the questionnaire score of at least 5 points compared to the baseline score, after 4 weeks of treatment;
- any changes in the GIS score (Global Index Score, calculated as the difference between the final score and the lowest possible score obtainable on the CIVIQ-20 questionnaire and divided by the difference between the theoretical maximum score and the minimum score (100-20=80), multiplied by (100) at the V2 visit and V3 compared with the GIS score obtained at baseline. The endpoint will be considered achieved if there is a reduction in the questionnaire score of at least 1 point from the baseline score after 4 weeks of treatment and after 12 weeks of treatment, respectively;
- any changes in the symptomatic 100-point VAS score at visit V2, V3 e V4 (see study design below) compared to the score obtained at baseline. The endpoint is considered reached in case of a reduction in the questionnaire score of at least 2 points compared to the baseline score, after 4 weeks of treatment, after 12 weeks of treatment and after 12 weeks of treatment plus 4 weeks of follow-up, respectively;
- any changes in the VDS score at visits V2, V3 e V4 compared to the score obtained at baseline. The endpoint is considered reached in case of a reduction in the questionnaire score of at least 1 point compared to the baseline score, after 4 weeks of treatment, after 12 weeks of treatment and after 12 weeks of treatment plus 4 weeks of follow-up, respectively;
- Percentage of subjects taking the product with adherence greater than or equal to 80% than the prescribed dosage regimen, noted at visits V2 and V3. Adherence is evaluated by counting the dosage units taken and by counting the dosage units returned or not used by the subjects and expressed as a percentage ratio;
- safety by assessing the incidence and type of any adverse events at each visit;
- tolerability of the product by assessing the occurrence of any side effects at visit V2, V3 e V4.

### Study population

The study protocol involves a total of 80 subjects of ≥ 18 years, of both sexes, with clinical symptoms compatible with CVD, who are not taking any medication and/or nutraceutical and/or a dietary supplement to treat the CVD from at least three months, who -once eligibility is confirmed and informed consent is signed- will be included in the study.

### Inclusion criteria

- Males and females;
- ≥ 18 years;
- Signature of the informed consent;
- Presence of at least one of the clinical symptoms possibly related to the chronic venous disease (feeling of heaviness and/or swelling and/or pain in the lower limbs, night cramps, feeling of tired/tired legs, itchy skin, burning and/or heat sensation, tingling, restlessness in the lower limbs);
- Availability to take part to the study and to adhere to the experimental procedure, attested by signing the written informed consent.

### Exclusion criteria

- Current pregnancy or breastfeeding;
- Women of childbearing potential who are not using adequate methods of birth control (whether hormonal in the form of the contraceptive pill or barrier method of birth control accompanied by the use of a spermicidal product foam / gel or film)

- Subjects with chronic venous insufficiency defined by C3-C6 stages according to CEAP classification
- Subjects presenting a positive foveal sign found at visit 1 after the signature of informed consent
- Patients with documented deep vein thrombosis for less than 6 months
- Intake in place or suspended for less than 4 weeks of venous protective and/or anti-edemigenous activity products
- Subjects eligible for surgery within three months of enrolment;
- Subjects with history of heart failure class NYHA III-IV, chronic renal insufficiency and /or sever hepatic insufficiency
- Ongoing neoplastic or immunosuppressive pathologies;
- Celiac disease
- Neurological pathologies or psychiatric conditions potentially capable of invalidating the consent and/or compromising the subject's adherence to the study procedures;
- Known allergy, hypersensitivity or intolerance to the constituents of the food supplement of the study
- Any condition, medical or otherwise, potentially capable - in the opinion of the Experimenter - of interfering with the study, or of advising against participation for safety reasons
- Subjects included in other clinical trials or to whom another experimental product has been administered during the 30 days before the beginning of the study
- Subjects who did not signed the informed content to the study.

### Drop out

Included subjects may drop out of the study at any time for the following reasons:
- Desire to leave the study;
- Refusal to cooperate with the study investigators;
- A medical condition that in the opinion of the investigator contraindicates the continuation of the study.

### Concomitant therapies

The study described is an interventional study involving the administration of a fixed combination of diosmin, hesperidin, bromelain, *Ananas comosus* extract and *Ruscus aculeatus* extract. The enrolled subjects will continue to follow the standard therapies (if in progress) required by their clinical situation. The therapies in progress for each subject will be noted in the study register.

Drugs (such as bioflavonoids, alpha benzopyrones) and/or nutraceuticals (saponins, citroflavonoids, triterpenes, polyphenols, anthocyanosides, etc.) used in the treatment of chronic venous disease must not be taken during the study period or in the 4 weeks prior to inclusion.

### Study design

The study is conducted according to an uncontrolled, polycentric, and competitive experimental design in subjects with clinical symptoms attributable to and/or suggestive of possible subsequent onset of chronic venous disease (C0s-C1s-C2s stages).

Each subject make 3 on-person visits (V1, V2, V3) and a telephone control (V4); V1 (screening; if positive, enrolment and admission to product intake), V2 (assessment after 4 weeks of taking the test product), V3 (assessment after 12 weeks of taking the test product; term intake), V4 (telephone interview after 16 weeks from V1), as reported in Figure 6.

### Statistical method and Expected Outcomes

Statistical analysis of the data is carried using the software GraphPad 8.0 (San Diego, California, US).

The main expected result is the efficacy of the test product (i.e. Vesvein) in bringing about an improvement in symptoms and thus quality of life associated with CVD, using the CIVIQ-20 test.

The template for informed consent is given to potential participants along with the information note.

This protocol is conducted in accordance with ethical principles that have their origin in the Declaration of Helsinki, comply with GCP and applicable regulatory provisions.

### References

1. Xu, S. Pharmacol Res 2020, 155, 104737. DOI: 10.1016/j.phrs.2020.104737.
3. Vanhoutte, P. M.; et al., Acta Physiol (Oxf) 2017, 219 (1), 22-96. DOI: 10.1111/apha.12646.
4. Hirase, T.; et al., Am J Physiol Heart Circ Physiol 2012, 302 (3), H499-505. DOI: 10.1152/ajpheart.00325.2011.
5. Castro-Ferreira, R.; et al., Ann Vasc Surg 2018, 46, 380-393. DOI: 10.1016/j.avsg.2017.06.131.
6. González-Pérez, F.; et al. Front Bioeng Biotechnol 2022, 10, 988533. DOI: 10.3389/fbioe.2022.988533.
7. Mansilha, A.; et al., Drug Therapy. Int J Mol Sci 2018, 19 (6). DOI: 10.3390/ijms19061669.
10. Casili, G.; et al., Vascul Pharmacol 2021, 137, 106825. DOI: 10.1016/j .vph.2020.106825.
12. Raffetto, J. D.; et al., Vessel Plus 2021, 5. DOI: 10.20517/2574-1209.2021.16.
13. Mannello, F.; et al., Curr Vasc Pharmacol 2013, 11 (3), 354-365. DOI: 10.2174/1570161111311030010.
14. Naik, B.; et al., Indian J Pathol Microbiol 2016, 59 (1), 25-30. DOI: 10.4103/0377-4929.178217.
15. Horecka, A.; et al., Ir J Med Sci 2021, 190 (1), 255-259. DOI: 10.1007/s11845-020-02289-1.
16. Kowalewski, R.; et al., Acta Angiol. 2011, 17, 141-149.
17. Serra, R.; et al., Nutrients 2021, 13 (3). DOI: 10.3390/nu13030999.
18. Maggioli, A. Phlebolymphology 2016, 23, 57-120.
19. Senra Barros, B.; et al., Int Angiol 2019, 38 (3), 211-218. DOI: 10.23736/S0392-9590.19.04116-6.
20. De Combarieu, E., et al., Fitoterapia, 73(7-8), 583-596.
21. Urbanek, T. Phlebological Review 2017, 25(1), 75-80.
22. Maurer, H. R. Cell Mol Life Sci 2001, 58 (9), 1234-1245. DOI: 10.1007/PL00000936.
23. Barakat, A.; et al., Dissolut. Technol. 2015, 22, 19-23.
24. Galla, R.; et al.,Biomedicines 2021, 9 (11). DOI: 10.3390/biomedicines9111543.
25. Uberti, F.; et al., Nutrients 2020, 12 (2). DOI: 10.3390/nu12020573.
26. Ceriotti, L.; et al., L'integratore Nutr. 2014, 17, 62-65.
27. Uberti, F.; et al., J Clin Endocrinol Metab 2014, 99 (4), 1367-1374. DOI: 10.1210/jc.2013-2103.
28. Felice, F.; et al., Phlebology 2018, 33 (9), 592-599. DOI: 10.1177/0268355517737662.
29. Galla, R.; et al., Int J Mol Sci 2022, 23 (15). DOI: 10.3390/ijms23158114
30. Fda.Gov.https://www.fda.gov/media/117974/download (accessed on 4 June 2024).
31. Ema.Eu. https://www.ema.europa.eu/en/documents/scientific-guideline/ich-m9-biopharmaceutics-classification-system-based-biowaivers-step-2b-first-version_en.pdf (accessed on 6 June 2024).
32. Chávez-García, D.; et al., J Biomed Mater Res B Appl Biomater 2020 108 (6), 2396-2406. https://doi.org/10.1002/jbm.b.34572.
33. Murphy, M. P.; et al., Nat metab, 2022, 4 (6), 651-662. https://doi.org/10.1038/s42255-022-00591-z.
34. Uberti, F.; et al., Nutrients 2017, 9 (9). DOI: 10.3390/nu9091008.
35. Guha, S.; et al., Nutrients 2021, 13 (5). DOI: 10.3390/nu13051448.
36. Konishi, Y.; et al., Biosci Biotechnol Biochem 2002, 66 (11), 2449-2457. DOI: 10.1271/bbb.66.2449.
37. Uberti, F.; et al., Int J Mol Sci 2024, 25 (4). DOI: 10.3390/ijms25042189.
38. Lee, S.; et al., J Biol Chem, 2015, 290 (2), 904-917. https://doi.org/10.1074/jbc.M114.606798.
39. Mulè, S.; et al., Oxid Med Cell Longev 2024, 2024, 7465045. DOI: 10.1155/2024/7465045.
40. Onodera, T.; et al., Sci rep 2024, 14(1), 8943. https://doi.org/10.1038/s41598-024-59685-1.
41. Velagapudi, R.; et al., Mol Nutr Food Res 2019, 63(10), e1801237. https://doi.org/10.1002/mnfr.201801237
42. Raffetto, J. D.; et al., Vessel Plus 2021, 5. DOI: 10.20517/2574-1209.2021.16.
43. Horecka, A.; et al., Ir J Med Sci 2021, 190 (1), 255-259. DOI: 10.1007/s11845-020-02289-1.
46. Belcaro, G.; et al., Int J Angiol 2017, 26 (3), 170-178. DOI: 10.1055/s-0036-1597756.
50. Raffetto, J.D.; et al., Int. Angiol. 2014, 33, 212-221.
51. Tong, N.; et al., PLoS One 2013, 8 (4), e61794. DOI: 10.1371/journal.pone.0061794.
53. Rajasekar et al. Carbohydrate Polymer Technologies and Applications 7 (2024) 100419
54. Pereira et al. Phytochem Rev (2024). https://doi.org/10.1007/s11101-024-10008-2
55. Sharma et al., Journal of Drug Delivery & Therapeutics. 2024; 14(5):148-157
56. Varilla et al. Foods 2021, 10, 2249. doi: 10.3390/foods10102249.
57. Greco M. et al., Phlebology 2023; 38 (5): 348-351
58. Jawien A. et al., Int Angio 2017; 36(1): 31-41
59. Allaert FA., Int Angio 2016; 35 (2):111-116
60. Fissore et al. Processes 2023, 11, 2064. https://doi.org/10.3390/pr11072064
61. Lurie F et al., J Vasc Surg Venous Lymphat Disord 2020;8(3):342-52.

## Claims

1. A composition comprising diosmin, hesperidin, bromelain, *Ruscus* extract and *Ananas* extract.

2. The composition according to claim 1 wherein diosmin is in an amount from 27% to 90% (w/w) of the total weight of the composition.

3. The composition according to claim 1 or 2 wherein hesperidin is in an amount from 3% to 10% (w/w) of the total weight of the composition.

4. The composition according to any of the preceding claims wherein bromelain is in an amount from 4% to 10% (w/w) of the total weight of the composition.

5. The composition according to claim 4 wherein the bromelain has an enzyme activity of at least 2500 GDU/g.

6. The composition according to any of the preceding claims wherein the *Ruscus* extract is in an amount from 1% to 6% (w/w) of the total weight of the composition.

7. The composition according to claim 6 wherein the *Ruscus* extract comprises from 5% to 15% of ruscogenin, preferably the *Ruscus* extract comprises 10% of ruscogenin.

8. The composition according to any of the preceding claims wherein the *Ananas* extract is in an amount from 0.1% to 2% (w/w) of the total weight of the composition.

9. The composition according to claim 8 wherein the *Ananas* extract comprises bromelain having an enzymatic activity of between 100 to 500 GDU/g, preferably the *Ananas* extract comprises bromelain having an enzymatic activity of 250 GDU/g.

10. The composition according to any of the preceding claims comprising about 44% (w/w) of diosmin, about 5% (w/w) of hesperidin, about 4% (w/w) of *Ruscus* extract, about 0.5% (w/w) of *Ananas* extract, and about 7.5% (w/w) bromelain of the total weight of the composition.

11. The composition according to any of preceding claims for use in the treatment and/or prevention of a venous disease or a venous insufficiency.

12. The composition for use according to claim 12 wherein the venous disease or the venous insufficiency is chronic venous disease or chronic venous insufficiency.

13. The composition for use according to any one of claim 11 or 12 wherein the chronic venous disease is varicose veins.

14. The composition according to any one of claim 11 to 13 for use in the treatment and/or prevention of venous ulcers, thrombus formation, painful oedema, swelling, and distal venous hypertension.

15. A food supplement or a nutritional composition comprising the composition according to any one of claim 1 to 10.
